# EUROPEAN PATENT SPECIFICATION

(11) **EP 3 840 767 B1**
(45) Date of publication and mention of the grant of the patent: **15.11.2023**
(21) Application number: 20728497.7
(22) Date of filing: 28.05.2020
(51) Int. Cl.: A61K 38/00, C07K 14/435, C07K 14/47, G01N 33/50

(54) **PEPTIDES**
PEPTIDE
PEPTIDES

(30) Priority: 29.05.2019 EP 19177444
(43) Date of publication of application: 30.06.2021
(60) Divisional application: 23185952.1
(73) Proprietor: Hubro Therapeutics AS, 0379 Oslo (NO)
(72) Inventor: ERIKSEN, Jon Amund, 3916 Porsgrunn (NO)
(74) Representative: Marks & Clerk LLP
(86) International application number: PCT/EP2020/064893
(87) International publication number: WO 2020/239937

(56) References cited:
- WO-A1-2018/223093
- WO-A2-99/58552
- P. MABY ET AL: "Correlation between Density of CD8+ T-cell Infiltrate in Microsatellite Unstable Colorectal Cancers and Frameshift Mutations: A Rationale for Personalized Immunotherapy", CANCER RESEARCH, vol. 75, no. 17, 1 September 2015 (2015-09-01), pages 3446-3455, XP055636602, US ISSN: 0008-5472, DOI: 10.1158/0008-5472.CAN-14-3051
- WESTDORP HARM ET AL: "Opportunities for immunotherapy in microsatellite instable colorectal cancer", CANCER IMMUNOLOGY, IMMUNOTHERAPY, NIH AUTHOR MANUSCRIPT, SPRINGER, BERLIN/HEIDELBERG, vol. 65, no. 10, 8 April 2016 (2016-04-08) , pages 1249-1259, XP036063227, ISSN: 0340-7004, DOI: 10.1007/S00262-016-1832-7 [retrieved on 2016-04-08]

## Description

### Field of Invention

The present invention provides peptides of TGBβR2 having a frameshift mutation for eliciting an immune response, peptide mixtures comprising peptides of TGBβR2 having a frameshift mutation for eliciting an immune response, non-transfected T-cells specific for such peptides, and T-cell mixtures comprising non-transfected T-cells specific for such peptides. The invention also relates to pharmaceutical formulations comprising such peptides, peptide mixtures, non-transfected T-cells and T-cell mixtures, uses of such peptides, peptide mixtures, non-transfected T-cells and T-cell mixtures for the prophylaxis and/or treatment of cancer, and methods of selecting peptides, peptide mixtures, non-transfected T-cells, T-cell mixtures for the treatment of cancer.

### Background

DNA microsatellites are strings of repetitive DNA, in which certain DNA motifs (nucleotide sequence patterns) are repeated, usually about 5 to 50 times. Microsatellite instability (MSI) is a change in the number of repeats of microsatellites and can be caused by impaired DNA mismatch repair (MMR) enzyme activity.

MMR corrects errors that occur spontaneously during DNA replication, such as single base mismatches or short insertions or deletions. When MMR activity is impaired, these spontaneous errors are not repaired, and this can result in microsatellite instability (i.e. a change in the number of repeats) and frameshift mutations in the DNA microsatellite sequences.

Frameshift mutations are the addition or deletion of one or two base pairs from a gene, resulting in different codons, and, therefore, a different protein being encoded, from the point of mutation. The frameshift typically results in truncated protein sequences because a STOP codon occurs prematurely, and the encoded proteins are usually defective or inactive.

In recent years, immuno-oncology has been a developing field, with efforts focussed on using the patient's own immune system to fight cancer. However, one problem is that antibodies can only bind to tumour antigens that are exposed on the surface of tumour cells. For this reason the efforts to produce a cancer treatment based on the immune system of the body has been less successful than expected.

TGFβR2 (SEQ ID NO: 1) is a growth factor, and its interaction with TGFβ mediates control of cell growth. Frameshift mutations in TGFβR2 render it biologically non-functional, thereby inducing uncontrolled cell growth and cancer progression. A single nucleotide deletion is by far the most dominant frameshift mutation in TGFβR2, although it is possible for a single nucleotide addition to occur. The amino acid sequence of TGFβR2 resulting from a single nucleotide deletion (-1a) frameshift mutation is shown in SEQ ID NO: 2.

The detection of MSI in cancer, such as colorectal cancers (CRCs), is performed by profiling the Bethesda panel, which is a reference panel including five microsatellite loci: two mononucleotides (BAT25 and BAT26) and three dinucleotides (D5S346, D2S123 and D17S250) (Cortes-Ciriano et al., Nature Communications, 2017, vol. 8, article no.15180; Vilar & Gruber, Nat Rev Clin Oncol, 2010, vol. 7(3), p.153-162). MSI is classed as high (MSI-H) when there is instability at two or more loci, and is classed as low (MSI-L) when there is instability at one locus (Vilar & Gruber, Nat Rev Clin Oncol, 2010, vol. 7(3), p.153-162). Microsatellites can be classed as stable (MSS) when there is no loci which has instabilities (Vilar & Gruber, Nat Rev Clin Oncol, 2010, vol. 7(3), p.153-162).

About 15% of all CRCs are MSI-H; Cortes-Ciriano et al., Nature Communications, 2017, vol. 8, article no.15180), and 99% of hereditary CRCs (Hereditary Non-Polyposis Colorectal Cancer (HNPCC)) are MSI-H (Pinheiro el al., British Journal of Cancer, 2015, vol. 113, p. 686-692). Of the MSI-H HNPCC patients, about 90% have a frameshift mutation in the protein TGFβR2 (Pinheiro el al., British Journal of Cancer, 2015, vol. 113, p. 686-692). People with HNPCC have a somatic mutation which is expected to develop into a frameshift mutation. CRC is often preceded by the development of polyps, but the removal of these from patients with hereditary CRC is ineffective in preventing cancer, unlike in patients who do not have hereditary CRC.

In addition, about 22% of stomach (gastric) cancers are MSI-H (Cortes-Ciriano et al., Nature Communications, 2017, vol. 8, article no.15180).

Furthermore, frameshift mutations in TGFβR2 are reported to be found in about 10% of all CRCs, about 44% of all MSI-H cancers, and in particular in about 58% of MSI-H colon cancers and about 80% of MSI-H stomach cancers (Cortes-Ciriano et al., Nature Communications, 2017, vol. 8, article no.15180).

Peptides of TGFβR2 having a frameshift mutation have been reported to be immunogenic, although there are inconsistencies in the results reported, as discussed further below.

EP1078000 discloses using fragments of proteins arising from frameshift mutations in the *BAX* and *TGFβR2* genes to treat cancer, by eliciting T-cell immunity.

Linnebacher et al. (Int J Cancer, 2001, 93, p.6-11) reports that three peptides derived from proteins having frameshift mutations were capable of activating specific CTLs (HLA-A2.1 restricted) *in vitro,* including a peptide (referred to therein as FSP02: RLSSCVPVA; SEQ ID NO: 10) of TGFβR2 having a -1a frameshift mutation. This peptide was also able to lyse the colorectal cancer cell line HCT116, which carries the corresponding frameshift mutation. However, two other peptides of -1a frameshifted TGFβR2 did not activate CTLs.

Saeterdal et al. (Cancer Immunol Immunother, 2001 Nov, 50(9), 469-476) reports that a peptide (RLSSCVPVA (labelled in Saeterdal et al., Cancer Immunol Immunother as p573); SEQ ID NO: 10 herein) of TGFβR2 having a frameshift mutation was able to generate a CTL line and several CTL clones. One CTL clone was able to kill an HLA-A2+ colon cancer cell line harbouring the frameshifted TGFβR2.

Saeterdal et al. (PNAS, 2001 Nov 6, 98(23), 13255-13260) reports a highly immunogenic peptide (labelled p538; SLVRLSSCVPVALMSAMTTSSSQ (SEQ ID NO: 11)) derived from TGFβR2 having a frameshift mutation, as a target for tumour infiltrating Th cells in MSI+ tumours. This peptide was recognized by two of three patients having spontaneous MSI+ colon cancer, and from all three patients with HNPCC. Several other peptides corresponding to the same frameshift mutation (p540: SPKCIMKEKKSLVRLSSCVPVA (SEQ ID NO: 12), and p541: PKCIMKEKKKSLVRLSSCV (SEQ ID NO: 13)) were also able to induce T-cell responses in some patients.

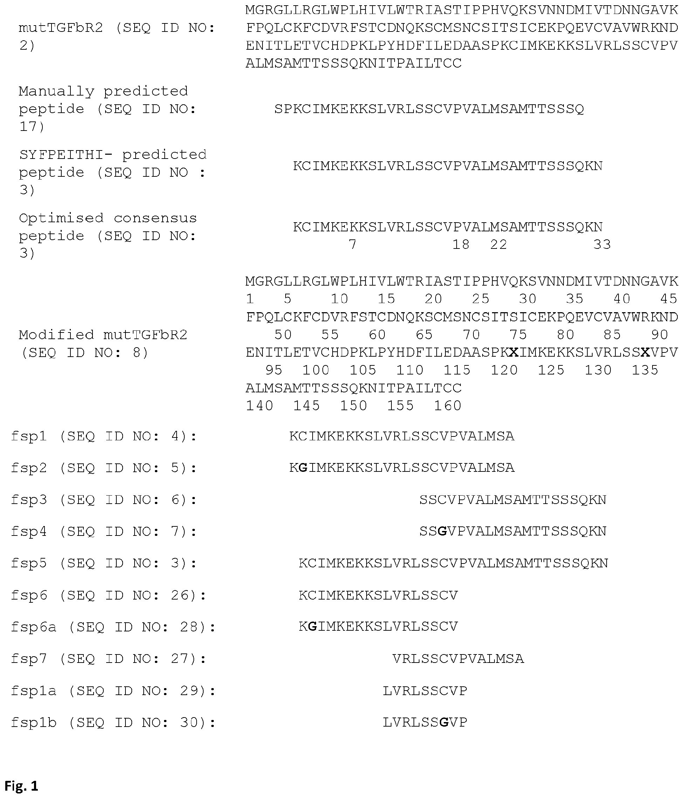

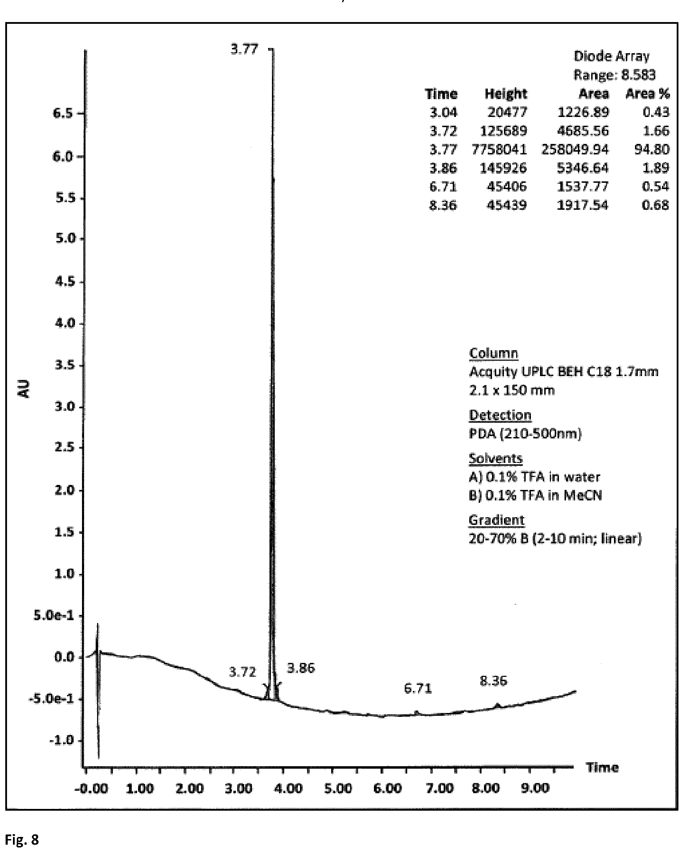

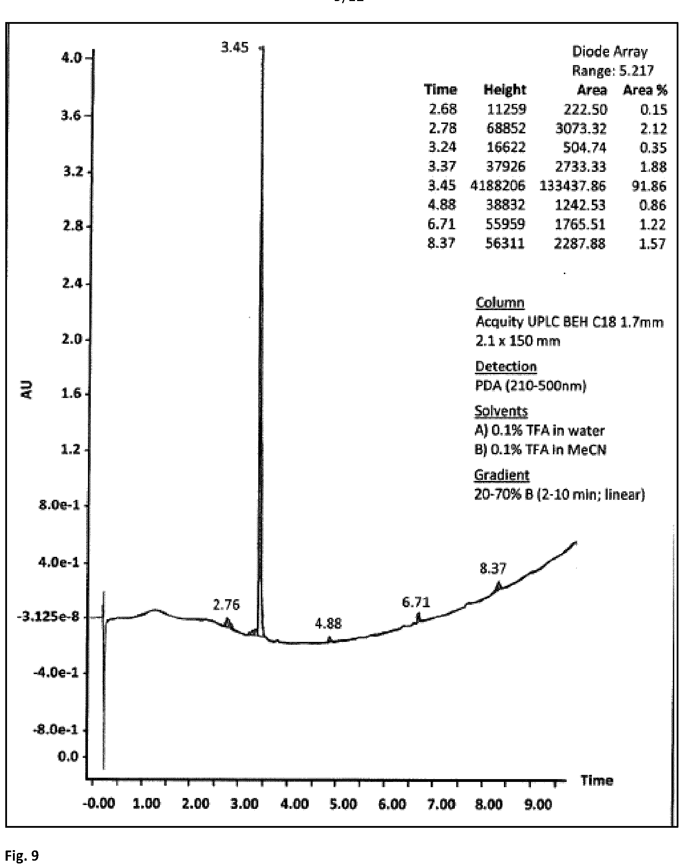

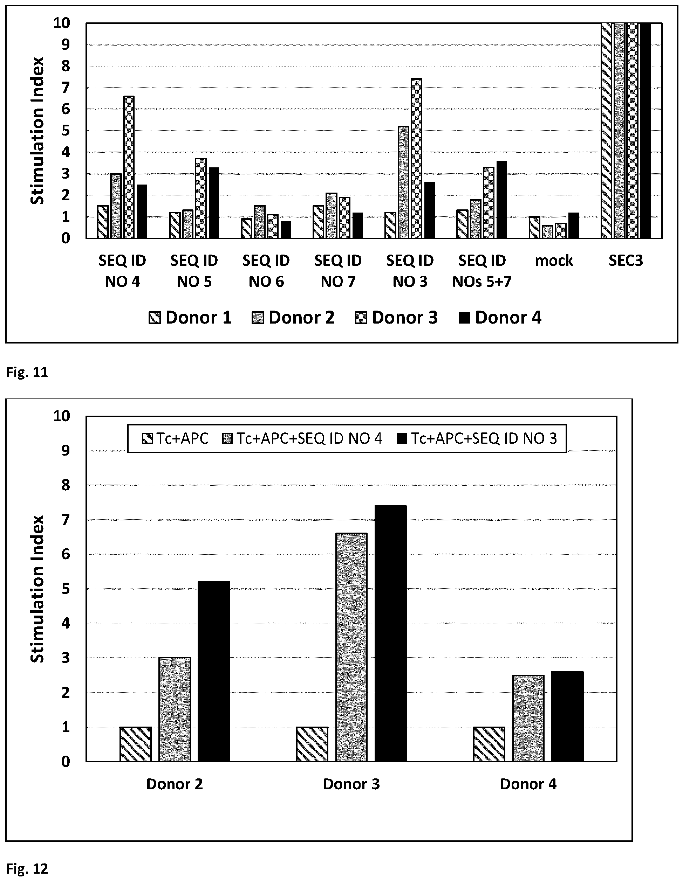

However, the studies above have contradictory results, such that it is not clear whether or not the peptides of TGFβR2 having a frameshift mutation are indeed immunogenic. For example, peptide FSP01 (SLVRLSSCV; SEQ ID NO: 13 herein) of Linnebacher *et al.* is the same as peptide SEQ ID NO: 428 of EP1078000, but Linnebacher *et al.* reports that this peptide is not immunogenic (Figure 1 of Linnebacher *et al.*) while EP1078000 reports that this peptide is immunogenic (Figure 14 of EP1078000), albeit only after four rounds of stimulation of the T-cells. Peptide FSP02 (RLSSCVPVA; SEQ ID NO: 10 herein) of Linnebacher *et al.* is the same as peptide SEQ ID NO: 439 of EP1078000 and peptide p573 of Saeterdal et al., (2001, Cancer Immunol Immunother), but Linnebacher *et al.* and Saeterdal et al (2001, Cancer Immunol Immunother) report that this peptide is immunogenic (Figure 1 of Linnebacher *et al.;* abstract, and page 472, column 1, paragraph 3, of Saeterdal et al.), while EP 1078000 reports that this peptide is not immunogenic even after four rounds of T-cell stimulation (Figure 14 of EP 1078000). Moreover, Saeterdal (2001, PNAS) discloses that the peptides p537 and p621 are not immunogenic but that the peptides p538, p540 and p4541 are immunogenic; however, both p538 and p621 comprise the sequence of peptides p523 and p573 which have been shown to be immunogenic by some studies (as discussed above). Thus, it is unclear why p621 is not immunogenic while p538 is immunogenic. Furthermore, EP1078000 discloses that the peptide SEQ ID NO: 17 thereof is capable of stimulating cultivated T-cell clones derived from a patient with adenocarcinoma (Figures 8 and 9 of EP1078000), but that the peptide SEQ ID NO: 17 thereof does not induce a T-cell response above background values in T-cells from healthy blood donors (Figure 12 of EP1078000). The results shown in Figures 8 and 9 of EP1078000 show that a spontaneous T-cell immune response might have been induced in a patient with cancer and that these T-cells, after cultivation with peptide SEQ ID NO: 17, can recognise peptide SEQ ID NO:17. However, these results do not show that the peptide SEQ ID NO: 17 is a strong enough antigen to induce a protective immune response.

US 8053552 discloses that a peptide derived from -1a frameshifted TGFβR2 was able, *in vitro,* to induce an immune response using T-cells from healthy HLA-A2.1+ donors. However, these results are limited only to HLA-A2.1+ epitopes, and do not show that other HLA class I-restricted T-cells, or any HLA class II-restricted T-cells, were induced. Historically, vaccines consisting only of HLA class I epitopes have not been successful in treating cancer and, therefore, US 8053552 does not show that the peptides tested therein are an effective vaccine or treatment for cancer.

WO 2018/223093 discloses peptides comprises a frameshift variant in a microsatellite coding region of an expressed gene, wherein the peptides are intended for use as a vaccine. The frameshift variant can be an addition or a deletion in the microsatellite, or can be downstream of a microsatellite. This document discloses the following peptides of mutated TGFβR2:
KKSLVRLSSCVPVALMSAMTTSSSQKNITPAILTCC (SEQ ID NO: 68) and
KEKKSLVRLSSCVPVALMSAMTTSSSQKNITPAILTCC (SEQ ID NO: 5986).

Thus, there is a need to provide effective vaccines and/or treatments for cancers, particularly cancers associated with MSI and frameshift mutations. In particular, there is a need to develop vaccines for people at risk of developing HNPCC. In addition, there is a need to provide vaccines and/or treatments for these cancers which are cost effective and can be used to treat or vaccine against as many MSI-H-associated cancers as possible.

### Summary of Invention

The present invention alleviates at least some of the problems above because it has now been found that peptides comprising a fragment of TGFβR2 having a frameshift mutation can be used to induce an immune response against cancer cells and, therefore, are useful for the treatment and/or prophylaxis of cancer associated with TGFβR2 having a frameshift mutation. The present invention is particularly useful for the treatment and/or prophylaxis of cancers associated with TGFβR2 having a -1a frameshift mutation (referred to herein as "mutTGFβR2"). It has been found that particularly useful peptides comprise a fragment of mutTGFβR2, and comprise a substitution of one amino acid of mutTGFβR2. In particular, peptides according to the present invention can be used to treat about 10% of all colorectal cancers, including about 90% of hereditary colorectal cancer, and about 18% of gastric cancers. The peptides of the present invention alleviate the issues of contradictory results for the immunogenicity of peptides of mutTGFβR2, and provide an effective and cost-effective vaccine and/or treatment. Moreover, the peptides of the present invention comprise multiple nested epitopes, such that the peptides comprise epitopes for more than one HLA allele. This provides the advantage that the peptides are capable of inducing an immune response in patients having different HLA alleles, such that the peptides are useful as a universal treatment and/or vaccine.

The invention is defined in the appended claims.

In a first aspect of the invention, there is provided a peptide comprising a fragment of SEQ ID NO: 8, wherein the fragment comprises at least 8 consecutive amino acids of SEQ ID NO: 8, including at least one of positions 121 and 135 of SEQ ID NO: 8, wherein the peptide is capable of inducing an immune response against a TGFβR2 -1a frameshift mutant protein

Preferably, there is provided a peptide comprising a fragment of SEQ ID NO: 8, wherein the fragment comprises at least 12 consecutive amino acids of SEQ ID NO: 8, including at least one of positions 121 and 135 of SEQ ID NO: 8, wherein the peptide is capable of inducing an immune response against a TGFβR2 -1a frameshift mutant protein.

Conveniently, the fragment comprises at least 9, preferably at least 12, consecutive amino acids of SEQ ID NO: 8, including at least one of positions 121 and 135 of SEQ ID NO: 8

Advantageously, the fragment comprises at least 15 consecutive amino acids of SEQ ID NO: 8, including at least one of positions 121 and 135 of SEQ ID NO: 8.

Conveniently, the fragment comprises only one of positions 121 and of SEQ ID NO: 8.

Advantageously, the peptide comprises no more than 33 amino acids.

Preferably, the peptide comprises no more than 24 amino acids.

Conveniently, the peptide comprises no more than 20 amino acids.

Preferably, the peptide comprises no more than 17 amino acids.

Conveniently, the peptide comprises no more than 9 amino acids.

Advantageously, the fragment comprises position 121 to 132 of SEQ ID NO: 8, positions 129 to 137 of SEQ ID NO: 8 or positions 135 to 146 of SEQ ID NO: 8.

Preferably, the amino acid corresponding to position 121 or 135 of SEQ ID NO: 8 is glycine.

Conveniently, outside of the fragment, the peptide has at least 70% sequence identity to SEQ ID NO: 8.

Advantageously, the peptide comprises the amino acid sequence of SEQ ID NO: 5, SEQ ID NO: 7, SEQ ID NO: 28 or SEQ ID NO: 30.

Preferably, the peptide consists of SEQ ID NO: 5, SEQ ID NO: 7, SEQ ID NO: 28 or SEQ ID NO: 30.

In some embodiments of the disclosure which are not part of the invention, the peptide further comprises a fragment of SEQ ID NO: 2, wherein the fragment comprises positions 6 to 13 of SEQ ID NO: 3, positions 7 to 22 of SEQ ID NO: 3, or positions 18 to 33 of SEQ ID NO: 3, wherein the peptide comprises no more than 40 amino acids, and wherein the peptide is capable of inducing an immune response against a TGFβR2 -1a frameshift mutant.

The disclosure includes a fragment which comprises i) positions 6 to 13 of SEQ ID NO: 3, and the peptide comprises no more than 21 amino acids; ii) positions 7 to 22 of SEQ ID NO: 3 and the peptide comprises no more than 40 amino acids; or iii) positions 18 to 33 of SEQ ID NO: 3, and the peptide comprises no more than 33 amino acids; wherein the peptide is capable of inducing an immune response against a TGFβR2 -1a frameshift mutant.

The fragment of the disclosure which is not part of the invention comprises positions 6 to 15 of SEQ ID NO: 3. Also disclosed herein, but not within the literal scope of the claims, is a peptide comprising a fragment of SEQ ID NO: 2, wherein the fragment comprises positions 2 to 22 of SEQ ID NO: 3 or positions 16 to 33 of SEQ ID NO: 3.

The peptide comprises the sequence of SEQ ID NO: 3.

In some instances of the disclosure, outside of the fragment, the peptide has at least 70% sequence identity to SEQ ID NO: 3.

In some instances of the disclosure, the peptide comprises the sequence of SEQ ID NO: 4, SEQ ID NO: 6 or SEQ ID NO: 26.

In some instances of the disclosure, the peptide consists of SEQ ID NO: 4, SEQ ID NO: 6 or SEQ ID NO: 26.

The peptide comprises no more than 33 amino acids, preferably no more than 24 amino acids, preferably no more than 20 amino acids or preferably no more than 17 amino acids.

The peptide which is not part of the invention i) comprises positions 6 to 13 of SEQ ID NO: 3 and comprises no more than 20 amino acids or, preferably, no more than 17 amino acids; ii) comprises positions 7 to 22 of SEQ ID NO: 3 and comprises no more than 33 amino acids, preferably no more than 24 amino acids, preferably no more than 20 amino acids or preferably no more than 17 amino acids; or iii) comprises positions 18 to 33 of SEQ ID NO: 3 and comprises no more than 24 amino acids, or preferably no more than 20 amino acids.

Also disclosed herein, but not part of the invention, is a peptide comprising a fragment of SEQ ID NO: 2, for use in the treatment and/or prophylaxis of cancer, wherein the fragment comprises the amino acid sequence of SEQ ID NO: 29, the peptide comprises no more than 21 amino acids and the peptide is capable of inducing an immune response against a TGFβR2 -1a frameshift mutant.

In some instances, the cancer is colorectal cancer or stomach cancer.

In some instances, the peptide comprises no more than 17 amino acids.

In a second aspect of the invention, there is provided a nucleic acid molecule encoding the peptide of the first aspect above.

In a third aspect of the invention, there is provided a peptide mixture comprising a first and a second peptide, wherein the first and second peptides are peptides according to the first aspect above, and wherein the first peptide is different from the second peptide.

In a fourth aspect of the invention, there is provided a vector comprising a nucleic acid molecule comprising a nucleotide sequence which encodes a peptide according to the first aspect above, or a peptide mixture according to the third aspect above.

In a fifth aspect of the invention, there is provided a host cell comprising a vector according to the fourth aspect above.

In a sixth aspect of the invention, there is provided a non-transfected T-cell specific for a peptide according to the first aspect above.

Also disclosed herein, but not within the literal scope of the claims, is a T-cell preparation comprising non-transfected T-cells specific for a peptide according to the first aspect, or the disclosures, above.

In a seventh aspect of the invention, there is provided a non-transfected T-cell mixture specific for a peptide mixture of the third aspect above.

In a eighth aspect of the invention, there is provided a pharmaceutical composition comprising a peptide according to the first aspect above, a nucleic acid molecule according to the second aspect above, a peptide mixture according to the third aspect above, a vector according to the fourth aspect above, a host cell according to the fifth aspect above, a non-transfected T-cell according to the sixth aspect above or a non-transfected T-cell mixture according to the seventh aspect above, and pharmaceutically-acceptable carrier, diluent or excipient.

In a ninth aspect of the invention, there is provided a peptide according to the first aspect above, a nucleic acid molecule according to the second aspect above, a peptide mixture according to the third aspect above, a vector according to the fourth aspect above, a host cell according to the fifth aspect above, a non-transfected T-cell according to the sixth aspect above, a non-transfected T-cell mixture according to the seventh aspect above or a pharmaceutical composition according to the eighth aspect above, for use in the treatment and/or prophylaxis of cancer.

Advantageously, the cancer is colorectal cancer or stomach cancer.

Conveniently, the cancer comprises cancer cells which express a frameshift mutant of the TGFβR2 protein.

Preferably, the frameshift mutant of the TGFβR2 protein is a -1a frameshift mutant.

In a tenth aspect of the invention, there is provided a method of selecting a peptide, nucleic acid molecule, peptide mixture, vector, host cell, T-cell, T-cell mixture or a pharmaceutical composition for administration to a patient, comprising:
i) identifying whether a cancer patient has a frameshift mutation in the TGFβR2 protein and, if so,
ii) selecting a peptide according to the first aspect above, a nucleic acid molecule according to the second aspect above, a peptide mixture according to the third aspect above, a vector according to the fourth aspect above, a host cell according to the fifth aspect above, a non-transfected T-cell according to the sixth aspect above, a non-transfected T-cell mixture according to the seventh aspect above or a pharmaceutical composition according to the eighth aspect above.

Also disclosed herein, but not within the literal scope of the claims, is a method of treating cancer comprising administering, to patient in need thereof, a peptide according to the first aspect, or as disclosed, above, a nucleic acid molecule according to the second aspect above, a peptide mixture according to the third aspect above, a vector according to the fourth aspect above, a host cell according to the fifth aspect above, a non-transfected T-cell according to the sixth aspect above, a non-transfected T-cell mixture according to the seventh aspect above or a pharmaceutical composition according to the eighth aspect above.

Also disclosed herein, but not within the literal scope of the claims, is the use of a peptide according to the first aspect, or as disclosed, above, a nucleic acid molecule according to the second aspect above, a peptide mixture according to the third aspect above, a vector according to the fourth aspect above, a host cell according to the fifth aspect above, a pharmaceutical composition according to the eighth aspect above comprising a peptide or peptide mixture, or a peptide, peptide mixture, nucleic acid molecule, vector, host cell or pharmaceutical composition for use according to the ninth aspect above, for the preparation of a non-transfected T-cell according to the sixth aspect above or non-transfected T-cell mixture according to the seventh aspect above.

The term "peptide", as used herein, refers to a polymer of amino acid residues that is (or has a sequence that corresponds to) a fragment of a longer protein. The term also applies to amino acid polymers in which one or more amino acid residues is a modified residue, or a non-naturally occurring residue, such as an artificial chemical mimetic of a corresponding naturally-occurring amino acid, as well as to naturally occurring amino acid polymers. The peptide may be linked to another agent or moiety.

The term "fragment", as used herein, refers to a series of consecutive amino acids from a longer polypeptide or protein.

The percentage "identity" between two sequences may be determined using the BLASTP algorithm version 2.2.2 Altschul, Stephen F., Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402), using default parameters. In particular, the BLAST algorithm can be accessed in the internet using the URL http://www.ncbi.nlm.nih.gov/blast/.

The term "immune response", as used herein, refers in some embodiments to a T-cell mediated immune response (i.e. T-cell activation) upon recognition of a peptide. The T-cell response may be a HLA-I mediated T-cell response and/or a HLA-II mediated T-cell response. The immune response may be a response by any alpha beta (αβ) T-cells and/or gamma delta (γδ) T-cells, such that the peptides may or may not be presented to the T-cells by major histocompatibility (MHC) molecules on the surface of antigen-presenting cells.

The term "frameshift mutant", as used herein, refers to a polypeptide encoded by a nucleic acid sequence having an addition or deletion of one or two nucleotides compared to the wild-type sequence of the nucleic acid, thereby resulting in different codons as of the point of mutation.

The term "-1a frameshift mutant", as used herein, refers to a polypeptide resulting from the deletion of a single nucleotide from the wild-type nucleic acid sequence.

The term "-1a frameshift mutation" refers to a change in the amino acid sequence of a polypeptide compared to the wild-type amino acid sequence of the polypeptide, resulting from the deletion of a single nucleotide from the nucleic acid sequence encoding that polypeptide.

The term "mutTGFβR2", as used herein, refers to a TGFβR2 protein which has a -1 a frameshift mutation. The amino acid sequence of mutTGFβR2 is shown in SEQ ID NO: 2.

The term "amino acid substitution", as used herein, refers to the replacement of an amino acid in a polypeptide with a different amino acid, compared to the wild-type amino acid sequence of the polypeptide.

The term "peptide mixture", as used herein, refers to two or more peptides which are mixed but not chemically combined. The mixtures may be present as a dry powder, solution, suspension or colloid, and may be homogeneous or heterogeneous.

The term "nucleic acid" or "nucleic acid molecule", as used herein, refers to a polymer of multiple nucleotides. The nucleic acid may comprise naturally occurring nucleotides or may comprise artificial nucleotides such as peptide nucleotides, morpholin and locked nucleotides as well as glycol nucleotides and threose nucleotides.

The term "nucleotide", as used herein, refers to naturally occurring nucleotides and synthetic nucleotide analogues that are recognised by cellular enzymes.

The term "pharmaceutical composition", as used herein, means a pharmaceutical preparation suitable for administration to an intended human or animal subject for therapeutic purposes.

### Brief Description of the Figures

Figure 1 shows the development of the consensus sequences, the peptides of the present invention and according to the disclosures herein, and a test peptide.
Figure 2 is a UPLC trace of freshly prepared crude fsp5 (SEQ ID NO: 3).
Figure 3 is a UPLC trace of purified fsp5 (SEQ ID NO: 3) in solution before lyophilisation.
Figure 4 is a UPLC trace of purified fsp5 (SEQ ID NO: 3) after lyophilisation.
Figure 5 is a HPLC trace of crude fsp5 (SEQ ID NO: 3) after storage for three days at room temperature, followed by reconstitution and lyophilisation.
Figure 6 is a UPLC trace of purified fsp1 (SEQ ID NO: 4).
Figure 7 is a UPLC trace of purified fsp2 (SEQ ID NO: 5).
Figure 8 is a UPLC trace of purified fsp3 (SEQ ID NO: 6).
Figure 9 is a UPLC trace of purified fsp4 (SEQ ID NO: 7).
Figure 10 is a graph showing T-cell proliferation after one round of stimulation with a peptide mixture containing fsp2 (SEQ ID NO: 5) and fsp4 (SEQ ID NO: 7).
Figure 11 is a graph showing T-cell proliferation after a second round of stimulation with a peptide mixture containing fsp2 (SEQ ID NO: 5) and fsp4 (SEQ ID NO: 7).
Figure 12 is a graph showing an alternative presentation of the T-cell proliferation in samples from Donors 2, 3, and 4 shown in Figure 11.
Figure 13 is a graph showing T-cell proliferation induced by fsp2 (SEQ ID NO: 5), fsp6 (SEQ ID NO: 26) or fsp7 (SEQ ID NO: 27), after stimulation with a peptide mixture containing fsp2 (SEQ ID NO: 5), fsp8 (SEQ ID NO: 31) and fsp9 (SEQ ID NO: 32).

### Brief Description of the Sequence Listing

SEQ ID NO: 1 is the full length wild-type TGFβR2 protein.
SEQ ID NO: 2 is the full-length mutated TGFβR2 with a -1 amino acid frameshift mutation.
SEQ ID NO: 3 is a 33-amino acid peptide of SEQ ID NO: 2, referred to herein as fsp5.
SEQ ID NO: 4 is a peptide referred to herein as fsp1.
SEQ ID NO: 5 is a peptide referred to herein as fsp2. Free text in sequence listing: modified peptide.
SEQ ID NO: 6 is a peptide referred to herein as fsp3.
SEQ ID NO: 7 is a peptide referred to herein as fsp4. Free text in sequence listing: modified peptide.
SEQ ID NO: 8 is the full-length mutated TGFβR2 with a -1 amino acid frameshift mutation and having amino acid substitutions at positions 121 and 135. Free text in sequence listing: Modified peptide; position 121 is any amino acid except cysteine; position 135 is any amino acid except cysteine.
SEQ ID NO: 9 is a prior art peptide of mutTGFβR2.
SEQ ID NO: 10 is a prior art peptide of mutTGFβR2.
SEQ ID NO: 11 is a prior art peptide of mutTGFβR2.
SEQ ID NO: 12 is a prior art peptide of mutTGFβR2.
SEQ ID NO: 13 is a prior art peptide of mutTGFβR2.
SEQ ID NO: 14 is a prior art peptide of mutTGFβR2.
SEQ ID NO: 15 is a prior art peptide of mutTGFβR2.
SEQ ID NO: 16 is a manually-predicted consensus sequence of mutTGFβR2.
SEQ ID NO: 17 is an epitope of mutTGFβR2 predicted by SYFPEITHI.
SEQ ID NO: 18 is an epitope of mutTGFβR2 predicted by SYFPEITHI.
SEQ ID NO: 19 is an epitope of mutTGFβR2 predicted by SYFPEITHI.
SEQ ID NO: 20 is an epitope of mutTGFβR2 predicted by SYFPEITHI.
SEQ ID NO: 21 is an epitope of mutTGFβR2 predicted by SYFPEITHI.
SEQ ID NO: 22 is an epitope of mutTGFβR2 predicted by SYFPEITHI.
SEQ ID NO: 23 is an epitope of mutTGFβR2 predicted by SYFPEITHI.
SEQ ID NO: 24 is an epitope of mutTGFβR2 predicted by SYFPEITHI.
SEQ ID NO: 25 is an epitope of mutTGFβR2 predicted by SYFPEITHI.
SEQ ID NO: 26 is a peptide referred to herein as fsp6.
SEQ ID NO: 27 is a peptide referred to herein as fsp7.
SEQ ID NO: 28 is a peptide referred to herein as fsp6a. Free text in sequence listing: Modified peptide.
SEQ ID NO: 29 is a peptide referred to herein as fsp1a.
SEQ ID NO: 30 is a peptide referred to herein as fsp1b. Free text in sequence listing: Modified peptide.
SEQ ID NO: 31 is a peptide referred to herein as fsp8.
SEQ ID NO: 32 is a peptide referred to herein as fsp9.

### Detailed Description

The invention relates, in general terms, to a peptide derived from TGFβR2 having a frameshift mutation. The peptide comprises a fragment of a TGFβR2 frameshift mutant protein and is able to induce an immune response against a TGFβR2 -1a frameshift mutant protein. The TGFβR2 frameshift mutant is a -1a TGFβR2 frameshift mutant (referred to herein as "mutTGFβR2"), and the amino acid sequence of the TGFβR2 -1a frameshift mutant protein is shown in SEQ ID NO: 2.

In some embodiments, the fragment of mutTGFβR2 (SEQ ID NO: 2) comprises at least 8, at least 9, at least 10, at least 12, at least 14, at least 15, at least 16, at least 18, at least 20, at least 22, at least 24, at least 26, at least 28, at least 30 or at least 32 amino acids. In some embodiments, the fragment comprises at least 20 amino acids. In other embodiments, the fragment comprises at least 24 amino acids. In further embodiments, the fragment comprises at least 33 amino acids.

In some embodiments, the fragment comprises no more than 100, 50 or 40 amino acids. For example, the fragment may comprise no more than 35, 33, 31, 29, 27, 25, 23, 21, 19, 17 or 9 amino acids. In some embodiments the fragment comprises no more than 33 amino acids. In other embodiments, the fragment comprises no more than 24, 20, 17 or 9 amino acids.

In some embodiments, the peptide comprises at least 8, at least 9, at least 10, at least 12, at least 14, at least 16, at least 18, at least 20, at least 22, at least 24, at least 26, at least 28, at least 30 or at least 32 amino acids. In some embodiments, the peptide comprises at least 20 amino acids. In other embodiments, the peptide comprises at least 24 amino acids. In some embodiments, the peptide comprises at least 17 amino acids. In other embodiments, the peptide comprises at least 9 amino acids. In further embodiments, the peptide comprises at least 33 amino acids.

In some embodiments, the peptide comprises no more than 100, 50 or 40 amino acids. For example, the peptide may comprise no more than 35, 33, 31, 29, 27, 25, 23, 21, 19, 17 or 9 amino acids. In some embodiments the peptide comprises no more than 33 amino acids. In other embodiments, the peptide comprises no more than 24, 20, 17 or 9 amino acids.

Thus, in some embodiments, the peptide comprises other amino acids outside of the fragment of mutTGFβR2. However, in other embodiments, the peptide is the same length as the fragment, such that the peptide is a fragment of mutTGFβR2.

### Sequence identity to mutTGFbR2 outside of the fragment

The peptide may have at least 70% sequence identity to mutTGFβR2 (SEQ ID NO: 2) outside of the fragment of mutTGFβR2. In some embodiments, the peptide has at least 75%, at least 80%, at least 85%, at least 90% or at least 95% sequence identity to SEQ ID NO: 2 outside of the fragment. In some embodiments, the peptide has 100% sequence identity to SEQ ID NO: 2 outside of the fragment of mutTGFβR2.

### Position of the fragment within the peptide

In some embodiments, the fragment of mutTGFβR2 (SEQ ID NO: 2) starts at position one, two, three, four, five, six or seven positions from the N-terminus of the peptide. In some embodiments, the fragment of mutTGFβR2 (SEQ ID NO: 2) ends at position one, two three, four, five, six, seven or eight from the C-terminus of the peptide. In other embodiments, the fragment is the C-terminus or the N-terminus of the peptide.

### Bridging region

In some embodiments, the fragment of mutTGFβR2 (SEQ ID NO: 2) comprises at least one amino acid from the wild-type sequence of TGFβR2 (SEQ ID NO: 1) (i.e. position 1 to position 127 of SEQ ID NO: 2) consecutive with at least one amino acid from the amino acid sequence resulting from the frameshift mutation (i.e. position 128 to position 161 of SEQ ID NO: 2). Thus, the fragment of mutTGFβR2 (SEQ ID NO: 2) may overlap the amino acid sequence unaffected by the frameshift mutation and the amino acid sequence resulting from the frameshift mutation. In some embodiments, the fragment comprises 1, 2, 3, 4, 5, 6, 7 or 8 consecutive amino acids from the wild-type amino acid sequence of TGFβR2 (SEQ ID NO: 1). In some embodiments, the at least one amino acid from the wild-type sequence of TGFβR2 (SEQ ID NO: 1) is consecutive with 1, 2, 3, 4, 5, 6, 7, 8, 9, 10, 11, 12, 13, 14, 15, 16, 17, 18, 19, 20, 21, 22, 23, 24 or 25 amino acids from the amino acid sequence resulting from the frameshift mutation.

In some embodiments, the fragment of mutTGFβR2 (SEQ ID NO: 2) comprises position 127 and position 128 of SEQ ID NO: 2, wherein the amino acid at position 127 of SEQ ID NO: 2 is the amino acid at position 127 of wild-type TGFβR2 (SEQ ID NO: 1). This corresponds to positions 8 and 9 of SEQ ID NO: 3, and positions 127 and 128 of SEQ ID NO: 8. In some embodiments, the fragment comprises position 126 to position 128, position 125 to position 128, position 124 to position 128, position 123 to position 128, position 122 to position 128, position 121 to position 128 or position 120 to position 128 of SEQ ID NO: 2 or SEQ ID NO: 8 (which correspond to position 7 to position 9, position 6 to position 9, position 5 to position 9, position 4 to position 9, position 3 to position 9, position 2 to position 9, or position 1 to position 9 of SEQ ID NO: 3, respectively).

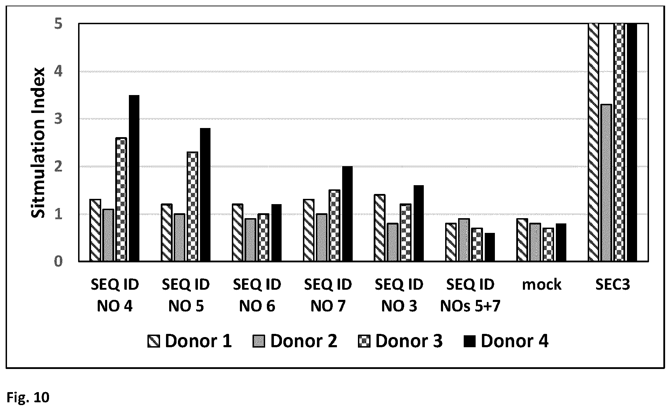

In particular, it is expected that at least the two amino acid residues bridging the wild-type amino acid sequence of TGFβR2 (SEQ ID NO: 1) and the amino acid sequence resulting from the frameshift mutation are helpful for providing an effective epitope, and for the peptide to have good immunogenicity. In particular, Figures 10 and 11 show that peptides comprising amino acids from the wild-type sequence of TGFβR2 (SEQ ID NO: 1) (i.e. fsp1 (SEQ ID NO: 4), fsp2 (SEQ ID NO: 5) and fsp5 (SEQ ID NO: 3)) are more immunogenic than peptides which do not comprise amino acids from the wild-type sequence of TGFβR2 (SEQ ID NO: 1) (i.e. fsp3 (SEQ ID NO: 6) and fsp4 (SEQ ID NO: 7)). It is also expected that the presence of more than one amino acid from the wild-type sequence of TGFβR2 (SEQ ID NO: 1), consecutive with at least one amino acid from the amino acid sequence resulting from the frameshift mutation, is likely to improve the immunogenicity of the peptide. In particular, it is expected that five, six, seven or eight amino acids from the wild-type sequence of TGFβR2 (SEQ ID NO: 1), consecutive with at least one amino acid from the amino acid sequence resulting from the frameshift mutation, will be particularly helpful to the immunogenicity of the peptide.

### Fsp1, fsp3, fsp5 & fsp6

In some embodiments, the fragment of mutTGFβR2 (SEQ ID NO: 2) further comprises positions 6 to 13 of SEQ ID NO: 3, positions 7 to 22 of SEQ ID NO: 3, or positions 18 to 33 of SEQ ID NO: 3. In this instance, the peptide comprises no more than 40 amino acids. In some embodiments, the fragment of mutTGFβR2 (SEQ ID NO: 2) comprises positions 6 to 13 of SEQ ID NO: 3 and the peptide comprises no more than 21 amino acids, the fragment comprises positions 7 to 22 of SEQ ID NO: 3 and the peptide comprises no more than 40 amino acids, or the fragment comprises positions 18 to 33 of SEQ ID NO: 3 and the peptide comprises no more than 33 amino acids. In some embodiments, the fragment comprises positions 6 to 15, positions 6 to 17, positions 2 to position 22 or positions 16 to position 33 of SEQ ID NO: 3. In some embodiments, the fragment comprises positions 6 to 15 of SEQ ID NO: 3, and the fragment starts at position six from the N-terminus of the peptide. In some embodiments, the fragment comprises positions 6 to 17 of SEQ ID NO: 3, and the fragment starts at position six from the N-terminus of the peptide. In some instances, which are not within the literal scope of the claims, the fragment comprises positions 2 to 15 of SEQ ID NO: 3, and the fragment starts at position two from the N-terminus of the peptide. In some instances, which are not within the literal scope of the claims, the fragment comprises positions 2 to position 22 of SEQ ID NO: 3, and the fragment starts at position two from the N-terminus of the peptide. In some instances, which are not within the literal scope of the claims, the fragment comprises position 1 to position 17, or position 1 to position 24, of SEQ ID NO: 3, and the fragment is the N-terminus of the peptide. In some instances, which are not within the literal scope of the claims, the fragment comprises position 16 to position 33 of SEQ ID NO: 3, and the fragment starts at position three from the N-terminus of the peptide. In some instances, which are not within the literal scope of the claims, the fragment comprises position 14 to position 33 of SEQ ID NO: 3. In some embodiments, the fragment comprises position 14 to position 33 of SEQ ID NO: 3, and the fragment is the N-terminus of the peptide.

In some instances, which are not part of the invention the fragment comprises the amino acid sequence of SEQ ID NO: 3, SEQ ID NO: 4, SEQ ID NO: 6 or SEQ ID NO: 26. In some instances, which are not within the literal scope of the claims, the fragment consists of the amino acid sequence of SEQ ID NO: 3, SEQ ID NO: 4, SEQ ID NO: 6 or SEQ ID NO: 26. When the peptide consists of the amino acid sequence of SEQ ID NO: 4, the peptide is referred to herein as "fsp1". When the peptide consists of the amino acid sequence of SEQ ID NO: 6, the peptide is referred to herein as "fsp3". When the peptide consists of the amino acid sequence of SEQ ID NO: 3, the peptide is referred to herein as "fsp5". When the peptide consists of the amino acid sequence of SEQ ID NO: 26, the peptide is referred to herein as "fsp6".

In some instances, which are not part of the invention the peptide comprises a fragment consisting of SEQ ID NO: 26 and the peptide comprises one or more additional amino acids at the C-terminus of the fragment. For example, the peptide may comprise one, two, three, four, five, six or seven additional amino acids at the C-terminus of the fragment. In these instances, the fragment consisting of SEQ ID NO: 26 may be the N-terminus of the peptide. Alternatively, the fragment consisting of SEQ ID NO: 26 may be the N-terminus of the peptide and the peptide may comprise seven additional amino acids at the C-terminus of the fragment. In another example, the fragment consisting of SEQ ID NO: 26 may be the N-terminus of the peptide and the peptide may consist of seven additional amino acids at the C-terminus of the fragment. The one or more additional amino acids may have at least 70%, at least 75%, at least 80%, at least 85%, at least 90%, at least 95%, or has 100%, sequence identity to the corresponding amino acids of SEQ ID NO: 2, and, preferably, may have 100% sequence identity to the corresponding amino acids of SEQ ID NO: 2.

### Fsp2, fsp4, fsp6a and fsp1b

The fragment of mutTGFβR2 (SEQ ID NO: 2) has an amino acid substitution compared to the naturally-occurring amino acid sequence of mutTGFβR2 (SEQ ID NO: 2). Thus, the fragment of mutTGFβR2 (SEQ ID NO: 2) comprises at least 8 consecutive amino acids of SEQ ID NO: 8, and includes at least one of positions 121 and 135 of SEQ ID NO: 8. In some embodiments the fragment may comprise at least 12 consecutive amino acids of SEQ ID NO: 8, and includes at least one of positions 121 and 135 of SEQ ID NO: 8. In some embodiments, the peptide comprises only one of positions 121 and 135 of SEQ ID NO: 8. In particular, positions 121 and 135 of SEQ ID NO: 8 correspond to positions 121 and 135 of mutTGFβR2 (SEQ ID NO: 2), respectively, which are both cysteine residues. The amino acid substitutions at positions 121 and 135 of SEQ ID NO: 8 are from cysteine to any other amino acid. Thus, the amino acid at positions 121 and 135 of SEQ ID NO: 8 is, independently, one of alanine, arginine, asparagine, aspartic acid, glutamine, glutamic acid, glycine, histidine, isoleucine, leucine, lysine, methionine, phenylalanine, proline, serine, threonine, tryptophan, tyrosine and valine. In some embodiments, the amino acid substitution is to glycine.

The amino acid substitution at position 121 and/or 135 of SEQ ID NO: 8, from cysteine to any other amino acid, is useful in preventing issues with production, stability, quality and immunology of the peptide. In particular, the peptides of the present invention are derived from the optimised consensus sequence (SEQ ID NO: 3), as discussed in the Examples and shown in Figure 1, and it has been found that the optimised consensus sequence can be difficult to synthesise due to its length. In addition, the solubility, stability and immunogenicity of the optimised consensus sequence (SEQ ID NO: 3) can be inadequate. In particular, the presence of one or more cysteine residues in a peptide can lead to molecular rearrangement and/or polymerisation of the peptide, due to the formation of inter- and/or intra-molecular disulphide bonds. This rearrangement and/or polymerisation may reduce the immunological potency of the peptide, and may induce unwanted inflammatory side effects through, for example, antibody formation and allergic reactions. The substitution of one or more cysteine residues in the peptide reduces the risk of these potential problems. Thus, in some embodiments, the substitution of one or more cysteine residues of the optimised consensus sequence improves the ease of production, the stability, the quality and the immunology of the peptide, but such substitutions are not essential for the present invention. Figure 1 shows how the peptides having the amino acid substitutions relate to the optimised consensus sequence.

### Range of positions for fsp2, fsp4, fsp6a and fsp1b

In some embodiments, the fragment of mutTGFβR2 (SEQ ID NO: 2) having an amino acid substitution comprises position 115 to position 122, position 116 to position 123, position 117 to position 124, position 118 to position 125, position 119 to position 126, position 120 to position 127, position 121 to position 128, position 128 to position 135, position 129 to position 136, position 130 to position 137, position 131 to position 138, position 132 to position 139, position 133 to position 140, position 134 to position 141 or position 135 to position 142 of SEQ ID NO: 8.

In some embodiments, the fragment of mutTGFβR2 (SEQ ID NO: 2) having an amino acid substitution comprises position 129 to position 137 of SEQ ID NO: 8.

In some embodiments, the fragment of mutTGFβR2 (SEQ ID NO: 2) having an amino acid substitution comprises position 115 to position 126, position 116 to position 127, position 117 to position 128, position 118 to position 129, position 119 to position 130, position 120 to position 131, position 121 to position 132, position 124 to position 135, position 125 to position 136, position 126 to position 137, position 127 to position 138, position 128 to position 139, position 129 to position 140, position 130 to position 141, position 131 to position 142, position 132 to position 143, position 133 to position 144, position 134 to position 145 or position 135 to position 146 of SEQ ID NO: 8. In some embodiments, the fragment of mutTGFβR2 (SEQ ID NO: 2) having an amino acid substitution comprises position 115 to position 129, position 116 to position 130, position 117 to position 131, position 118 to position 132, position 119 to position 133, position 120 to position 134, position 121 to position 135, position 122 to position 136, position 123 to position 137, position 124 to position 138, position 125 to position 139, position 126 to position 140, position 127 to position 141, position 128 to position 142, position 129 to position 143, position 130 to position 144, position 131 to position 145, position 132 to position 146, position 133 to position 147, position 134 to position 148 or position 135 to position 149 of SEQ ID NO: 8. In some embodiments, the fragment of mutTGFβR2 (SEQ ID NO: 2) comprises position 119 to position 130, position 119 to position 133, position 120 to position 131, position 120 to position 134, position 121 to position 132, position 121 to position 135, position 133 to position 144, position 133 to position 147, position 134 to position 145, position 134 to position 148, position 135 to position 146, or position 135 to position 149 of SEQ ID NO: 8.

In some embodiments, the fragment of mutTGFβR2 (SEQ ID NO: 2) having an amino acid substitution consists of position 115 to position 122, position 115 to position 126, position 115 to position 129, position 116 to position 123, position 116 to position 127, position 116 to position 130, position 117 to position 124, position 117 to position 128, position 117 to position 131, position 118 to position 125, position 118 to position 129, position 118 to position 132, position 119 to position 126, position 119 to position 130, position 119 to position 133, position 120 to position 127, position 120 to position 131, position 120 to position 134, position 121 to position 128, position 121 to position 132, position 121 to position 135, position 122 to position 136, position 123 to position 137, position 124 to position 135, position 124 to position 138, position 125 to position 136, position 125 to position 139, position 126 to position 137, position 126 to position 140, position 127 to position 138, position 127 to position 141, position 128 to position 135, position 128 to position 139, position 128 to position 142, position 129 to position 136, position 129 to position 140, position 129 to position 143, position 130 to position 137, position 130 to position 141, position 130 to position 144, position 131 to position 138, position 131 to position 142, position 131 to position 145, position 132 to position 139, position 132 to position 143, position 132 to position 146, position 133 to position 140, position 133 to position 144, position 133 to position 147, position 134 to position 141, position 134 to position 145, position 134 to position 148, position 135 to position 142, position 135 to position 146 or position 135 to position 149 of SEQ ID NO: 8.

### fsp2 and fsp6a (and fsp5 with a C-to-G substitution)

In some embodiments, the fragment of mutTGFβR2 (SEQ ID NO: 2) comprises position 121, but not position 135, of SEQ ID NO: 8. In some embodiments, the peptide comprises at least 70%, at least 75%, at least 80%, at least 85%, at least 90%, at least 95%, or has 100%, sequence identity to SEQ ID NO: 2 outside of the fragment. In some such embodiments, the peptide comprises 100% sequence identity to SEQ ID NO: 2 outside of the fragment. In some embodiments, the peptide comprises no more than 33 amino acids, no more than 24 amino acids or no more than 17 amino acids. In some embodiments, the peptide consists of 33 amino acids. In other embodiments, the peptide consists of 24 amino acids. In other embodiments, the peptide consists of 17 amino acids. In some embodiments, the fragment of mutTGFβR2 (SEQ ID NO: 2) comprises position 119 to position 126, position 120 to position 127 or position 121 to position 128 of SEQ ID NO: 8. In some embodiments, the fragment of mutTGFβR2 (SEQ ID NO: 2) comprises position 119 to position 130, position 120 to position 131 or position 121 to position 132 of SEQ ID NO: 8. In other embodiments, the fragment comprises position 119 to position 133, position 120 to position 134 or position 121 to position 135 of SEQ ID NO: 8. In some embodiments, the fragment consists of position 119 to position 130, position 119 to position 133, position 120 to position 131, position 120 to position 134, position 121 to position 132 or position 121 to position 135 of SEQ ID NO: 8. In some embodiments, the fragment of mutTGFβR2 (SEQ ID NO: 2) having an amino acid substitution starts at position one, two or three from the N-terminus of the peptide. In some embodiments, the fragment is the N-terminus of the peptide. In some embodiments, position 121 of SEQ ID NO: 8 is glycine.

In some embodiments, the peptide comprises a fragment of mutTGFβR2 (SEQ ID NO: 2) having an amino acid substitution wherein the fragment consists of position 119 to position 126, position 120 to position 127 or position 121 to position 128 of SEQ ID NO: 8, wherein position 121 of SEQ ID NO: 8 is glycine, and wherein the peptide has 100% sequence identity to SEQ ID NO: 2 outside of the fragment and comprises no more than 33 amino acids. In some embodiments, the peptide comprises a fragment of mutTGFβR2 (SEQ ID NO: 2) having an amino acid substitution wherein the fragment consists of position 119 to position 130, position 119 to position 133, position 120 to position 131, position 120 to position 134, position 121 to position 132 or position 121 to position 135 of SEQ ID NO: 8, wherein position 121 of SEQ ID NO: 8 is glycine, and wherein the peptide has 100% sequence identity to SEQ ID NO: 2 outside of the fragment and comprises no more than 33 amino acids. In some embodiments, the fragment is the N-terminus of the peptide. In some such embodiments, the peptide consists of 33 amino acids. In other embodiments, the peptide consists of 24 amino acids, and the peptide may consist of the amino acid sequence of SEQ ID NO: 5. Peptides consisting of the amino acid sequence of SEQ ID NO: 5 are referred to herein as "fsp2". In other embodiments, the peptide consists of 17 amino acids, and the peptide may consist of the amino acid sequence SEQ ID NO: 28. Peptides consisting of the amino acid sequence of SEQ ID NO: 28 are referred to herein as "fsp6a".

In some embodiments, the peptide comprises a fragment consisting of SEQ ID NO: 28 and the peptide comprises one or more additional amino acids at the C-terminus of the fragment. For example, the peptide may comprise one, two, three, four, five, six or seven additional amino acids at the C-terminus of the fragment. In some embodiments, the fragment consisting of SEQ ID NO: 28 is the N-terminus of the peptide. In some embodiments, the fragment consisting of SEQ ID NO: 28 is the N-terminus of the peptide and the peptide comprises seven additional amino acids at the C-terminus of the fragment. In other embodiments, the fragment consisting of SEQ ID NO: 28 is the N-terminus of the peptide and the peptide consists of seven additional amino acids at the C-terminus of the fragment. In some embodiments, the one or more additional amino acids have at least 70%, at least 75%, at least 80%, at least 85%, at least 90%, at least 95%, or has 100%, sequence identity to the corresponding amino acids of SEQ ID NO: 2, and, preferably, have 100% sequence identity to the corresponding amino acids of SEQ ID NO: 2.

### Fsp4 and fsp1b

In some embodiments, the fragment of mutTGFβR2 (SEQ ID NO: 2) having an amino acid substitution comprises position 135, but not position 121, of SEQ ID NO: 8. In some embodiments, the peptide comprises at least 70%, at least 75%, at least 80%, at least 85%, at least 90%, at least 95%, or has 100%, sequence identity to SEQ ID NO: 2 outside of the fragment. In some embodiments, the peptide comprises no more than 20 amino acids, while in other embodiments the peptide consists of 20 amino acids. In some embodiments, the peptide comprises no more than 9 amino acids, while in other embodiments the peptide consists of 9 amino acids.

In some embodiments, the fragment of mutTGFβR2 (SEQ ID NO: 2) comprises position 129 to position 136, position 130 to position 137, position 133 to position 140, position 134 to position 141 or position 135 to position 142 of SEQ ID NO: 8. In some embodiments, the fragment of mutTGFβR2 (SEQ ID NO: 2) comprises position 129 to position 137, position 133 to position 144, position 134 to position 145 or position 135 to position 146 of SEQ ID NO: 8. In some embodiments, the fragment of mutTGFβR2 (SEQ ID NO: 2) having an amino acid substitution comprises position 133 to position 147, position 134 to position 148 or position 135 to position 149 of SEQ ID NO: 8. In some embodiments, the fragment of mutTGFβR2 (SEQ ID NO: 2) having an amino acid substitution consists of position 129 to position 136, position 130 to position 137, position 133 to position 144, position 133 to position 147, position 134 to position 145, position 134 to position 148, position 135 to position 146 or position 135 to position 149 of SEQ ID NO: 8. In some embodiments, the fragment of mutTGFβR2 (SEQ ID NO: 2) having an amino acid substitution starts at position one, two or three from the N-terminus of the peptide. In some embodiments, the fragment of mutTGFβR2 (SEQ ID NO: 2) having an amino acid substitution is the N-terminus of the peptide. In some embodiments, position 135 of SEQ ID NO: 8 is glycine.

In some embodiments, the peptide comprises a fragment of mutTGFβR2 (SEQ ID NO: 2) having an amino acid substitution, wherein the fragment consists of the amino acid sequence of SEQ ID NO: 30. In some embodiments, the peptide consists of the amino acid sequence of SEQ ID NO: 30, and such peptides are referred to herein as "fsp1b".

In some embodiments, the peptide comprises a fragment of mutTGFβR2 (SEQ ID NO: 2) having an amino acid substitution wherein the fragment consists of position 133 to position 140, position 133 to position 144, position 133 to position 147, position 134 to position 141, position 134 to position 145, position 134 to position 148, position 135 to position 142, position 135 to position 146 or position 135 to position 149 of SEQ ID NO: 8, wherein position 135 of SEQ ID NO: 8 is glycine, and wherein the peptide has 100% sequence identity to SEQ ID NO: 2 outside of the fragment and comprises no more than 20 amino acids. In some embodiments, the fragment is the N-terminus of the peptide. In some embodiments, the peptide consists of 20 amino acids. In some embodiments, the peptide consists of the amino acid sequence of SEQ ID NO: 7, and such peptides are referred to herein as "fsp4".

### Fsp1a

Also disclosed herein, but not within the literal scope of the claims, are peptides of mutTGFβR2 (SEQ ID NO: 2) for use in the treatment and/or prophylaxis of cancer, wherein the peptide comprises a fragment of SEQ ID NO: 2, wherein the fragment comprises the amino acid sequence of SEQ ID NO: 29 and wherein the peptide comprises no more than 21 amino acids.

These peptides of mutTGFβR2 (SEQ ID NO: 2) may comprise no more than 17 amino acids. In some instances, the peptide comprises no more than 9 amino acids. In other instances, the peptide consists of 21, 17 or 9 amino acids. The peptide may comprise at least 70%, at least 75%, at least 80%, at least 85%, at least 90%, at least 95% or at least 100% sequence identity to SEQ ID NO: 2 outside of the fragment.

In some instances, the fragment consists of the sequence of SEQ ID NO: 29.

In some instances, the peptide consists of the amino acid sequence of SEQ ID NO: 29, and such peptides are referred to herein as "fsp1a".

As discussed in Example 8, it is expected that the amino acid sequence of SEQ ID NO: 29 is immunogenic in view of the difference in immunogenicity of the amino acid sequences of SEQ ID NO: 26 and SEQ ID NO: 27, and the similarities and differences between these amino acid sequences. Similarly, it is expected that the amino acid sequence of SEQ ID NO: 30, which is identical to SEQ ID NO: 29 except for a C-to-G amino acid substitution, is also immunogenic, as it has been shown that a C-to-G substitution is immunologically acceptable.

The peptides correspond to mutTGFβR2 (SEQ ID NO: 2) fragments displayed by MHC I or MHC II molecules on the surface of cells and/or to which individuals generally have a reactive T-cell in their T-cell repertoire. The peptide is able to induce an immune response against a TGFβR2 -1a frameshift mutant protein and, preferably, the immune response is a T-cell response, comprising both MHC-I-restricted T-cells, such as CD8+ T-cells, and MHC-II-restricted T-cells, such as CD4+ T-cells. In particular, the peptides may encompass multiple nested epitopes, such that each peptide may comprise epitopes for more than one MHC allele. This provides the advantage that the peptides are capable of inducing an immune response in patients having different MHC alleles, such that the peptides are useful as a universal treatment and/or vaccine.

### Peptide mixture

The invention also provides mixtures of the above-described peptides of the invention, wherein the peptide mixture comprises two or more different peptides. The peptide mixtures of the present invention comprise a first and a second peptide, wherein the first and the second peptide are peptides of the invention, as described above, and the first peptide is different from the second peptide. In some embodiments, the first peptide comprises a fragment of mutTGFβR2 (SEQ ID NO: 2) which comprises positions 6 to 13 of SEQ ID NO: 3, comprises positions 7 to 22 of SEQ ID NO: 3, comprises positions 18 to 33 of SEQ ID NO: 3, comprises at least 8 or at least 12 consecutive amino acids of SEQ ID NO: 8 including at least one of positions 121 and 135 of SEQ ID NO: 8. In some instances which are not within the literal scope of the claims, the first peptide comprises a fragment of mutTGFβR2 (SEQ ID NO: 2) which comprises at least 8 consecutive amino acids of SEQ ID NO: 29, and the second peptide, independently, comprises a fragment of mutTGFβR2 (SEQ ID NO: 2) which comprises positions 6 to 13 of SEQ ID NO: 3, comprises positions 7 to 22 of SEQ ID NO: 3, comprises positions 18 to 33 of SEQ ID NO: 3, comprises at least 8 or at least 12 consecutive amino acids of SEQ ID NO: 8 including at least one of positions 121 and 135 of SEQ ID NO: 8, or comprises at least 8 consecutive amino acids of SEQ ID NO: 29, wherein the second peptide is different from the first peptide. In some instances which are not within the literal scope of the claims, the first peptide comprises a fragment of mutTGFβR2 (SEQ ID NO: 2) wherein the fragment comprises positions 6 to 13 of SEQ ID NO: 3, comprises positions 7 to 22 of SEQ ID NO: 3, comprises positions 18 to 33 of SEQ ID NO: 3 or comprises at least 8 consecutive amino acids of SEQ ID NO: 8 including at least one of positions 121 and 135 of SEQ ID NO: 8, and the second peptide is a peptide for use in treatment and/or prophylaxis of cancer as described above, wherein the fragment of mutTGFβR2 (SEQ ID NO: 2) comprises at least 8 consecutive amino acids of SEQ ID NO: 29. In some embodiments, each of the first and second peptides are independently selected from a peptide comprising a fragment of mutTGFβR2 (SEQ ID NO: 2) wherein the fragment comprises positions 6 to 13 of SEQ ID NO: 3, comprises positions 7 to 22 of SEQ ID NO: 3, comprises positions 18 to 33 of SEQ ID NO: 3, or comprises at least 8 consecutive amino acids of SEQ ID NO: 8 including at least one of positions 121 and 135 of SEQ ID NO: 8.

In some embodiments, the peptide mixture comprises a first peptide and a second peptide, wherein the first peptide comprises a fragment of SEQ ID NO: 8 which comprises position 121 but not position 135 of SEQ ID NO: 8, and the second peptide comprises a fragment of SEQ ID NO: 8 which comprises position 135 but not position 121 of SEQ ID NO: 8. In some embodiments, the first peptide comprises no more than 33, 24 or 17 amino acids and has 100% sequence identity to SEQ ID NO: 2 outside of the fragment. In some embodiments, the fragment of SEQ ID NO: 8 of the first peptide comprises, or may consist of, position 119 to position 126, position 119 to position 130, position 119 to position 133, position 120 to position 127, position 120 to position 131, position 120 to position 134, position 121 to position 128, position 121 to position 132 or position 121 to position 135 of SEQ ID NO: 8. In some embodiments, the first peptide comprises a glycine residue at position 121 of SEQ ID NO: 8. In some embodiments, the second peptide comprises no more than 20 amino acids and has 100% sequence identity to SEQ ID NO: 2 outside of the fragment. In some embodiments, the fragment of SEQ ID NO: 8 of the second peptide comprises, and, preferably, consists of, position 129 to position 136, position 130 to position 137, position 129 to position 137, position 133 to position 140, position 133 to position 144, position 133 to position 147, position 134 to position 141, position 134 to position 145, position 134 to position 148, position 135 to position 142, position 135 to position 146 or position 135 to position 149 of SEQ ID NO: 8. In some embodiments, the second peptide comprises a glycine residue a position 135 of SEQ ID NO: 8.

In some embodiments, the first and second peptides, independently, consist of the amino acid sequence of SEQ ID NO: 5, SEQ ID NO: 7, SEQ ID NO: 28or SEQ ID NO: 30. In some instances which are not within the literal scope of the claims, the first and second peptides, independently, consist of the amino acid sequence of SEQ ID NO: 3, SEQ ID NO: 4, SEQ ID NO: 6. SEQ ID NO: 26 or SEQ ID NO: 29. In some embodiments, the first peptide consists of the amino acid sequence of SEQ ID NO: 5, and the second peptide consists of the amino acid sequence of SEQ ID NO: 7.

In other embodiments, the first peptide consists of the amino acid sequence of SEQ ID NO: 5 and the second peptide consists of the amino acid sequence of SEQ ID NO: 7. In particular, Figure 11 shows that some donors showed a higher response to a mixture of SEQ ID NO: 5 and SEQ ID NO: 7 (i.e. fsp2 and fsp4) than to SEQ ID NO: 5 or SEQ ID NO: 7 alone, after two rounds of stimulation.

The peptide mixtures may comprise one or more further peptides, in addition to the first and second peptides, which may be one or more peptides as described above. The one or more further peptides are different from each of the first and the second peptides. Where the peptide mixture comprises more than one further peptide, the further peptides are different from each other.

The peptide mixtures may contain the peptides in equal or different proportions. In some embodiments, the first and second peptides are present in the mixture in equal proportions, i.e. each peptide comprises 50% of the peptide component of the peptide mixture. In other embodiments, there is a greater proportion of the first peptide in the peptide mixture than the second peptide. For example, the first peptide may comprise at least 55%, at least 60%, at least 70%, at least 80% or at least 90% of the peptide component of the peptide mixture. In alternative embodiments, there is a greater proportion of the second peptide in the peptide mixture than the first peptide. For example, the second peptide may comprise at least 55%, at least 60%, at least 70%, at least 80% or at least 90% of the peptide component of the peptide mixture. In embodiments comprising at least one further peptide, the peptides are present in the peptide component of the peptide mixture in equal proportions. In other embodiments, the first, second and the at least one further peptide are present in different proportions from each other. For example, each of the first, second and at least one further peptide may independently comprise at least 1%, at least 5%, at least 10%, at least 20% at least 30%, at least 40%, at least 50%, at least 60%, at least 60%, at least 70%, at least 80% or at least 90% of the peptide component of the peptide mixture.

### Nucleic acids

In another aspect of the present invention, there is provided a nucleic acid molecule which comprises a nucleotide sequence which encodes a peptide, or a peptide of a peptide mixture, of the invention. Also disclosed herein, but not within the literal scope of the claims, is a nucleic acid molecule which comprises a nucleotide sequence which encodes a peptide, or a peptide of a peptide mixture, according to the disclosures above. In some embodiments, the nucleic acid molecule comprises a nucleotide sequence which encodes at least two peptides of the peptide mixture. There is also provided a mixture of nucleic acid molecules, wherein each nucleic acid molecule of the mixture comprises a nucleotide sequence which encodes a different peptide of a peptide mixture according to the disclosures above or of the invention, such that the mixture of nucleic acid molecules encodes the peptide mixture of the disclosures above or of the invention. In some embodiments of the mixture of nucleic acid molecules, each nucleic acid molecule comprises a nucleotide sequence which encodes at least two peptides of the peptide mixture.

In some embodiments, the nucleic acid molecules and mixtures of nucleic acid molecules are used to synthesise the peptides and peptides mixtures of the disclosures above. For example, a peptide of the disclosures above may be synthesised by administering a nucleic acid molecule to a subject, whereupon the nucleic acid molecule is expressed by the subject, thereby giving rise to a peptide of the disclosures above *in situ.* The peptide produced then elicits an immune response in the subject. In another example, the nucleic acid molecule may be used to synthesise a peptide of the disclosures above, *in vitro,* by transforming or transfecting a host cell with the nucleic acid molecule, such that the host cell expresses the nucleic acid molecule to produce the peptide of the disclosures above. The peptide is then recovered and purified. In some embodiments, the peptides of the disclosures above are produced by chemical synthesis, using methods well known in the art.

### T-cells & T-cell mixtures

In another aspect of the present invention, there is provided a non-transfected T-cell, specific for a peptide of the invention. Also disclosed herein, but not within the literal scope of the claims, is a non-transfected T-cell preparation comprising one or more non-transfected T-cells specific for a peptide, or a peptide for use, according to the disclosures above. There is further provided a non-transfected T-cell mixture comprising non-transfected T-cells specific for each of the peptides in one of the peptide mixtures of the invention or of the disclosures above.

The non-transfected T-cell, non-transfected T-cell preparation and non-transfected T-cell mixture may be ex *vivo* and may be produced by stimulating, ex *vivo,* at least one reactive non-transfected T-cell with a peptide or a peptide mixture according to the disclosures above. For example, in one embodiment, the non-transfected T-cell is specific for a peptide comprising a fragment of SEQ ID NO: 8, wherein the fragment comprises at least 8 or at least 12 consecutive amino acids of SEQ ID NO: 8, including at least one of position 121 and position 135 of SEQ ID NO: 8. In another embodiment, the non-transfected T-cell is specific for a peptide comprising a fragment of mutTGFβR2 (SEQ ID NO: 2), wherein the fragment comprises positions 6 to 13, positions 7 to 22 of SEQ ID NO: 3, or positions 18 to 33 of SEQ ID NO: 3, wherein the peptide comprises no more than 40 amino acids. In some instances which are not within the literal scope of the claims, the non-transfected T-cell is specific for a peptide comprising a fragment of mutTGFβR2 (SEQ ID NO: 2), wherein the fragment comprises the amino acid sequence of SEQ ID NO: 29 and the peptide comprises no more than 21 amino acids. In another embodiments, the non-transfected cell is specific for a peptide comprising a fragment of mutTGFβR2 (SEQ ID NO: 2), wherein the fragment comprises positions 6 to 13 of SEQ ID NO: 3 and the peptide comprises no more than 21 amino acids; or the fragment comprises positions 7 to 22 of SEQ ID NO: 3 and the peptide comprises no more than 40 amino acids; or the fragment comprises positions 18 to 33 of SEQ ID NO: 3 and the peptide comprises no more than 33 amino acids. In some instances which are not within the literal scope of the claims the non-transfected T-cell preparation comprises one or more non-transfected T-cells specific for a peptide comprising a fragment of mutTGFβR2 (SEQ ID NO: 2), wherein the fragment comprises an amino acid substitution compared to SEQ ID NO: 2 and comprises at least 8 or at least 12 consecutive amino acids of SEQ ID NO: 8, including at least one of position 121 and position 135 of SEQ ID NO: 8; or wherein the fragment comprises positions 6 to 13 of SEQ ID NO: 3 or positions 7 to 22 of SEQ ID NO: 3, and the peptide comprises no more than 40 amino acids; or wherein the fragment comprises positions 18 to 33 of SEQ ID NO: 3 and the peptide comprises no more than 40 amino acids; or wherein the fragment comprises the amino acid sequence of SEQ ID NO: 29 and the peptide comprises no more than 21 amino acids. In some instances which are not within the literal scope of the claims, the non-transfected T-cell preparation comprises one or more non-transfected T-cells specific for a peptide comprising a fragment of mutTGFβR2 (SEQ ID NO: 2), wherein the fragment comprises an amino acid substitution compared to SEQ ID NO: 2 and comprises at least 8 consecutive amino acids of SEQ ID NO: 8, including at least one of position 121 and position 135 of SEQ ID NO: 8; or wherein the fragment comprises positions 6 to 13 of SEQ ID NO: 3 and the peptide comprises no more than 21 amino acids; or wherein the fragment comprises positions 7 to 22 of SEQ ID NO: 3 and the peptide comprises no more than 40 amino acids; or wherein the fragment comprises positions 18 to 33 of SEQ ID NO: 3 and the peptide comprises no more than 33 amino acids, or wherein the fragment comprises the amino acid sequence of SEQ ID NO: 29 and the peptide comprises no more than 21 amino acids. In another embodiment, the non-transfected T-cell mixture comprises a plurality of non-transfected T-cells, wherein a first and a second non-transfected T-cell are specific for a first and a second peptide of mutTGFβR2 (SEQ ID NO: 2) according to the disclosures above, respectively, wherein the first peptide is different from the second peptide.

Where the T-cell receptor of any T-cell disclosed herein is an αβ T-cell receptor, then the T-cell receptor is specific for the peptide when presented on an MHC molecule. Where the T-cell receptor of any T-cell disclosed herein is a γδ T-cell receptor, then the T-cell receptor does not necessarily require presentation of the peptide on an MHC molecule in order to recognise the peptide.

### Vector & host cell

In another aspect of the present invention, there is provided a vector comprising a nucleic acid molecule comprising a nucleotide sequence which encodes a peptide or a peptide mixture of the invention. Also disclosed herein, but not within the literal scope of the claims, is a vector comprising a nucleic acid molecule comprising a nucleotide sequence which encodes a peptide, peptide for use or a peptide mixture of the disclosures above. In some embodiments, the vector is a DNA vector or a RNA vector.

In a further aspect, there is provided a host cell comprising a vector of the invention. Also disclosed herein, but not within the literal scope of the claims, is a host cell comprising a vector according to the disclosures above. The host cell is transfected or transformed with the vector, such that the host cell expresses the nucleic acid molecule(s) encoded by the vector. The host cell may be any cell type that is capable of being transfected with a vector and expressing the vector. In some embodiments, the host cell is a plant cell, an animal cell, a micro-organism, or a yeast cell. In some embodiments, the host cell is a dendritic cell.

### Pharmaceutical compositions

Pharmaceutical compositions comprising the peptides, peptide mixtures, non-transfected T-cells, non-transfected T-cell mixtures, nucleic acid molecules, vectors, or host cells of the invention are also provided. Also disclosed herein, but not within the literal scope of the claims, are pharmaceutical compositions comprising peptides, peptide mixtures, non-transfected T-cells, non-transfected T-cell mixtures, non-transfected T-cell preparations, nucleic acid molecules, vectors or host cells described above. Such pharmaceutical compositions may also comprise at least one pharmaceutically acceptable carrier, diluent and/or excipient. In some embodiments, the pharmaceutical composition further comprises one or more additional active ingredients and/or adjuvants. In certain embodiments, the pharmaceutical composition may further comprise one or more ingredients therapeutically effective for the same disease indication. In one embodiment, the pharmaceutical composition of the present invention may further comprise one or more further chemotherapeutic agents, one or more cancer vaccines, one or more antibodies, one or more small molecules and/or one or more immune stimulants (for example, cytokines). In some embodiments, the peptide, peptide mixture, non-transfected T-cell, non-transfected T-cell mixture, nucleic acid, vector, host cell or the pharmaceutical composition may be used in combination with other forms of immunotherapy, including other cancer vaccines. In some instances, which are not within the literal scope of the claims, the non-transfected T-cell preparations may be used in combination with other forms of immunotherapy, including other cancer vaccines. In some embodiments, the peptide, peptide mixture, non-transfected T-cell, non-transfected T-cell mixture, nucleic acid, vector, host cell or the pharmaceutical composition is used in combination with one or more cancer vaccines derived from a different cancer antigen. In some instances, which are not within the literal scope of the claims, the non-transfected T-cell preparations may be used in one or more cancer vaccines derived from a different cancer antigen.

### Use

Peptides, peptide mixtures, non-transfected T-cells, non-transfected T-cell preparations, non-transfected T-cell mixtures, nucleic acid molecules, vectors, host cells and pharmaceutical composition of the invention and disclosed above are for use in the treatment and/or prophylaxis of cancer, and in particular cancers associated with a frameshift mutation, preferably a -1a frameshift mutation, in TGFβR2. In particular, about 10% of all CRCs, and about 44% of all MSI-H cancers, have a frameshift mutation in TGFβR2. The cancer may be colorectal cancer or stomach cancer. The colorectal cancer may be colon cancer or rectal cancer. The peptides, peptide mixtures, non-transfected T-cells, non-transfected T-cell preparations, non-transfected T-cell mixtures, nucleic acid molecules, vectors and host cells may be used for the treatment and/or prophylaxis of more than one of these types of cancer. In particular, the peptides, peptide mixtures, non-transfected T-cells, non-transfected T-cell preparations, non-transfected T-cell mixtures, nucleic acid molecules, vectors and host cells of the disclosures above can be used to treat about 10% of all colorectal cancers (i.e. all hereditary and spontaneous CRCs), including about 85.5% of all hereditary colorectal cancers, and about 18% of all stomach cancers. Thus, the present invention provides an effective treatment for a large proportion of cancers, particularly colorectal cancer and stomach cancer, and more particularly, hereditary colorectal cancer.

In some embodiments, a peptide comprising a fragment of SEQ ID NO: 8, wherein the fragment comprises at least 8 or at least 12 consecutive amino acids of SEQ ID NO: 8, including position 121 of SEQ ID NO: 8, wherein the peptide comprises no more than 33 amino acids, is particularly useful for the treatment of cancer. In some embodiments, the peptide comprises a fragment of SEQ ID NO: 8, wherein the fragment comprises at least 8 or at least 12 consecutive amino acids of SEQ ID NO: 8, including position 121, but not position 135, of SEQ ID NO: 8, wherein the amino acid at position 121 of SEQ ID NO: 8 is glycine, and the peptide has 100% sequence identity to SEQ ID NO: 2 outside of the fragment of mutTGFβR2 (SEQ ID NO: 2). In some embodiments the peptide consists of 17, 24 or 33 amino acids, and the fragment of SEQ ID NO: 8 consists of 8, 10, 12 or 15 consecutive amino acids of SEQ ID NO: 8. In some embodiments, the peptide consists of the amino acid sequence of SEQ ID NO: 5. In particular, it has been found that the peptide consisting of the amino acid sequence of SEQ ID NO: 5 (i.e. fsp2) is particularly useful as a vaccine or treatment against cancer because it is completely unknown to the human immune system and is able to induce an immune response not only to itself but also to the naturally-occurring corresponding sequence (i.e. SEQ ID NO: 4; fsp1), as shown in Figure 11. In other words, fsp2 is cross-reactive for the naturally-occurring mutTGFβR2. Figure 11 shows the results of stimulation of donors with a peptide mixture consisting of SEQ ID NO: 5 and SEQ ID NO: 7 (i.e. fsp2 and fsp4), and Donor 3, for example, showed an immune response to the peptide of SEQ ID NO: 4 (i.e. fsp1; a fragment of naturally-occurring mutTGFβR2) as well as SEQ ID NO: 5 (i.e. fsp2).

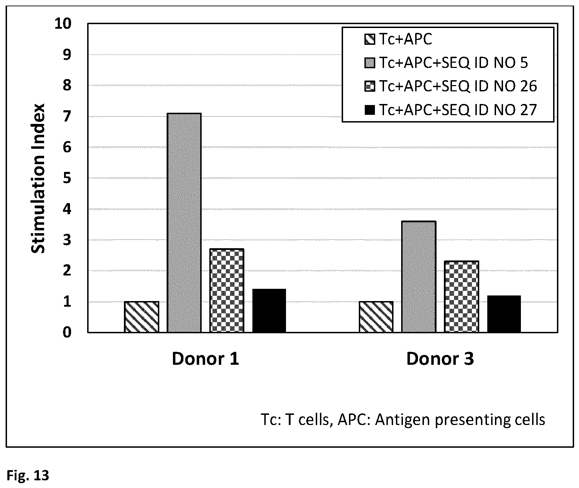

The peptides of SEQ ID NOs: 3, 4, 7, 26, 28, 29 and 30 are also particularly useful as a vaccine or treatment against cancer. Figures 11 and 12 show that T-cells induced from PBMCs using a mixture of SEQ ID NO: 5 (i.e. fsp2) and SEQ ID NO: 7 (i.e. fsp4) are stimulated by SEQ ID NO: 3 (i.e. fsp 5), SEQ ID NO: 4 (i.e. fsp1) and SEQ ID NO: 7 (i.e. fsp4), as well as SEQ ID NO: 5 (i.e. fsp2), meaning that these peptides are also immunogenic and are useful as a vaccine or treatment against cancer. Moreover, Figure 13 shows that T-cells induced from PMBCs using the peptide of SEQ ID NO: 5 (i.e. fsp2) are stimulated by the peptide of SEQ ID NO: 26 (i.e. fsp6), such that fsp6 (SEQ ID NO: 26) can also be used as a vaccine or treatment against cancer.

Figure 13 also shows that fsp6a (SEQ ID NO: 28), which has a single C-to-G amino acid substitution compared to fsp6 (SEQ IDNO: 26), is expected to be immunogenic in view of Figure 11, which shows that both fsp1 and fsp2 are immunogenic. As mentioned above, fsp2 (SEQ ID NO: 5) is able to induce an immune response both to itself and to the naturally-occurring corresponding sequence (SEQ ID NO: 4, fsp1), showing that the C-to-G amino acid substitution in fsp2 compared to fsp1 is immunologically acceptable. It is therefore expected that a corresponding C-to-G amino acid substitution in fsp6 (as shown in fsp6a) would not compromise the immunogenicity of such a modified peptide.

In addition, Figure 13 shows that the full immunogenic activity of fsp2 (SEQ ID NO: 5) can be achieved by adding at least one amino acid to the C-terminus of fsp6 (SEQ ID NO: 26) or fsp6a (SEQ ID NO: 28), up to the full length of fsp2 (SEQ ID NO: 5; i.e. up to an additional seven amino acids at the C-terminus). Moreover, Figure 13 shows that fsp7 (SEQ ID NO: 27) is too truncated at the N-terminus compared to fsp2 (SEQ ID NO 5) to be immunogenic. Therefore, in order for a peptide to be immunogenic against mutTGFβR2, it must comprise at least one additional amino acid at the N-terminus of fsp7 (SEQ ID NO: 27).

Furthermore, and as discussed above, Figure 13 shows that it is expected that fsp1a (SEQ ID NO: 29) and fsp1b (SEQ ID NO: 30) are immunogenic in view of the difference in immunogenicity of the amino acid sequences of SEQ ID NO: 26 and SEQ ID NO: 27, and the similarities and differences between these amino acid sequences.

The non-transfected T-cell, or the non-transfected T-cells in the T-cell preparation or T-cell mixture, for use in the treatment and/or prophylaxis of cancer may be autologous or allogenic. For example, heterologous T-cells may be administered to a patient where the T-cells are from a donor having the same or similar HLA repertoire as the patient.

The peptide, peptide mixture, vector, host cell or pharmaceutical composition of the invention or according to the disclosures above, or the peptide for use according to the disclosures above, may be administered to a subject by any suitable delivery technique known to those skilled in the art. For example, among other techniques, the peptide, peptide mixture or pharmaceutical composition may be administered to a subject by injection, in the form of a solution, in the form of liposomes or in dry form (for example, in the form of coated particles, etc). The host cell may be administered, for example, by transfusion. The vector may be administered, for example, by injection subcutaneously or into the tumour. In some embodiments, the peptide, peptide mixture or pharmaceutical composition may be administered in an amount, for example, of between 1µg and 1g of each peptide once every three days, once a week, once a month, once every three months, once every four months or once every six months. In some embodiments, the net amount of each peptide per dose is 60nM. For example, for intradermal injection, each peptide may be present in a volume of 0.1ml at a concentration of 0.6mM.

In some embodiments, the peptide or peptide mixture is administered with an adjuvant or immune stimulator, such as GM-CSF. In embodiments using GM-CSF, this may be any GM-CSF, for example, glycosylated GM-CSF or non-glycosylated GM-CSF. GM-CSF may be administered in an amount of between 0.5 and 120 µg/ m², between 1 and 120 µg/ m², between 2 and 115 µg/ m², between 3 and 110 µg/ m², between 4 and 105 µg/ m², between 5 and 100 µg/ m², between 6 and 95 µg/ m², between 7 and 90 µg/ m², between 48 and 85 µg/ m², between 9 and 80 µg/ m², between 10 and 75 µg/ m², between 11 and 70 µg/ m², between 12 and 65 µg/ m², between 13 and 60 µg/ m², between 14 and 55 µg/ m², between 15 and 50 µg/ m², between 16 and 45 µg/ m², between 17 and 40 µg/ m², or between 18 and 40 µg/ m² of body surface area. In some embodiments, GM_CSF is administered at a dosage of between 1 µg and 200 µg, between 5 µg and 175 µg, between 5 µg and 150 µg, between 5 µg and 125 µg, between 5 µg and 100 µg, between 10 µg and 100 µg, between 20 µg and 90 µg, between 25 µg and 80 µg, between 25 µg and 70 µg, between 25 µg and 65 µg, or between 30 µg and 60 µg, per dose. In some embodiments, non-glycosylated GM-CSF is administered at a dosage of 30µg per dose. In other embodiments, glycosylated GM-CSF is administered at a dosage of 60µg dose. In embodiments where GM-CSF is administered by intradermal injection, the dose may be a 0.1ml solution containing GM-CSF at a concentration of 0.3mg/ml or 0.6mg/ml. In some embodiments, the peptide, peptide mixture or pharmaceutical composition may be administered in an amount, for example, of between 1µg and 1g of each peptide once every three days, once a week, once a month, once every three months, once every four months or once every six months.

The non-transfected T-cells, non-transfected T-cell mixtures and non-transfected T-cell preparations of the present invention and according to the disclosures above which are not within the literal scope of the claims may be administered by intra-venous injection and/or infusion, and may be administered in an amount, for example, of between 10⁶ and 10¹² of each non-transfected T-cell specific for a peptide of the peptide mixture or peptide once every month, once every two months, once every three months, once every six months or once a year. Preferably, the dosage is administered once every month for between 2 and 5 months and is subsequently administered less frequently.

The nucleic acid and mixture of nucleic acids of the present invention and according to the disclosures herein may be administered by intra-muscular injection and/or subcutaneous injection.

Administration of a peptide, a peptide for use or a peptide mixture of the present invention and according to the disclosures herein to a subject, or expression of the peptide or peptide mixture by a subject, elicits an immune response to the peptide or peptide mixture, in particular a T-cell mediated immune response. The peptide, or each peptide of the peptide mixture, may be processed by an antigen-presenting cell (APC) and may be presented on an MHC molecule. αβ T-cells are activated by binding of the T-cell receptor to a peptide presented on a MHC molecule by the APC, thereby resulting in an immune response against tumour cells having a mutation corresponding to that present in the administered peptide(s). γδ T-cells do not necessarily require antigen processing or presentation of the antigen by MHC molecules.

In another aspect of the invention, there is provided a peptide, peptide mixture, non-transfected T-cell, non-transfected T-cell mixture, nucleic acid molecule or a pharmaceutical composition for use in a method of comprising the diagnosis of cancer and the selection of an appropriate treatment. Also disclosed herein, but not within the literal scope of the claims, is a non-transfected T-cell preparation for use in a method of comprising the diagnosis of cancer and the selection of an appropriate treatment. In some instances which are not within the literal scope of the claims, the method comprises the steps of (i) identifying whether a cancer patient is MSI-H and, if so, (ii) selecting a peptide, peptide mixture, non-transfected T-cell, non-transfected T-cell preparation, non-transfected T-cell mixture, nucleic acid molecule or pharmaceutical composition according to the disclosures above. The method of the invention comprises, (i), testing whether the patient has a frameshift mutation in the TGFβR2 protein and, if so, (ii) selecting a peptide, peptide mixture, non-transfected T-cell, non-transfected non-transfected T-cell mixture, nucleic acid molecule or pharmaceutical composition according to the disclosures above. In some embodiment, the frameshift mutation is a -1a frameshift mutation. In some instances which are not within the literal scope of the claims, the method further comprises (iii) administering the selected peptide, peptide mixture, T-cell, T-cell preparation, non-transfected T-cell mixture, nucleic acid molecule or pharmaceutical composition to the patient. It is envisaged that any of the peptides, peptide mixture, non-transfected T-cells, non-transfected T-cell preparations, non-transfected T-cell mixtures, nucleic acid molecules or pharmaceutical compositions disclosed above can be used to treat cancers associated with MSI-H, more specifically cancers associated with a -1a frameshift mutation in TGFβR2, because the peptides all correspond to the same protein.

Also disclosed herein, but not within the literal scope of the claims, is a method of treating and/or preventing cancer comprising administering a peptide, peptide mixture, non-transfected T-cell, non-transfected T-cell preparation, non-transfected T-cell mixture, nucleic acid molecule or pharmaceutical composition according to the disclosures above to a patient in need thereof. The method may comprise the steps of (i) identifying a cancer patient as MSI-H, and (ii) administering a peptide, peptide mixture, non-transfected T-cell, non-transfected T-cell preparation, non-transfected T-cell mixture, nucleic acid molecule or pharmaceutical composition according to the disclosures above to the patient. The method may further comprise, in step (i), the step of identifying that the patient has a frameshift mutation in the TGFβR2 protein. In some instances, the frameshift mutation may be a -1a frameshift mutation.

Also disclosed herein, but not within the literal scope of the claims, is a kit that includes a peptide, a peptide for use, a peptide mixture, a non-transfected T-cell, a non-transfected T-cell mixture, a non-transfected T-cell preparation, a nucleic acid molecule, a nucleic acid molecule mixture, a vector and/or a host cell of the invention or according to the disclosures above. The peptide, peptide for use, a peptide mixture, a non-transfected T-cell, a non-transfected T-cell mixture, a non-transfected T-cell preparation, a nucleic acid, a nucleic acid mixture, a vector and/or a host cell as such may be present in the kit, or the peptide, peptide for use, a peptide mixture, a non-transfected T-cell, a non-transfected T-cell mixture, a non-transfected T-cell preparation, a nucleic acid molecule, a nucleic acid mixture, a vector and/or a host cell may be present as a pharmaceutical formulation. In some instances, the peptide, peptide for use, peptide mixture, non-transfected T-cell, non-transfected T-cell preparation, non-transfected T-cell mixture, nucleic acid molecule mixture, vector and/or host cell may be packaged, for example in a vial, bottle, flask, which may be further packaged, for example, within a box, envelope or bag. In some instances, the kit comprises a peptide mixture, a non-transfected T-cell mixture and/or nucleic acid molecule mixture wherein the peptides, the non-transfected T-cells and/or the nucleic acid molecules are provided in separate containers, such that the peptides, non-transfected T-cells and/or nucleic acid molecules are mixed by the user.

### Examples

### Example 1

Previous studies on peptides for use as a vaccine against cancers associated with TGFbR2 having a frameshift mutation showed that at least some peptides may be immunogenic, but there are contradictory results. Thus, a consensus peptide was manually predicted based upon the peptides tested in earlier studies.

**Table 1: Previously tested peptides of mutTGFβR2 and their T-cell activation result**

| **Previously Tested Peptide Sequence** | **T cell activation** | **Peptide label** |
|---|---|---|
| SPKCIMKEKKSLVRLSSCVPVA (SEQ ID NO: 11) | + | P540 |
| SLVRLSSCVPVALMSAMTTSSSQ (SEQ ID NO: 10) | + | p538 |
| SLVRLSSCV (SEQ ID NO: 13) | + | p523 |
| RLSSCVPVA (SEQ ID NO: 9) | + | p573 |
| ALMSAMTTSSSQKNITPAILTCC (SEQ ID NO: 14) | - | p539 |
| AMTTSSSQKNITPAILTCC (SEQ ID NO: 15) | - | p537 |

Predicted consensus sequence: SPKCIMKEKKSLVRLSSCVPVALMSAMTTSSSQ (SEQ ID NO: 16).

### Example 2

An online algorithm (i.e. SYFPEITHI) was then used to predict epitopes of mutTGFβR2 for HLA class II alleles. The HLA class II alleles included in the search were: HLA-DRB1*0101, HLA-DRB1*0301 (DR17), HLA-DRB1*0401 (DR4Dw4), HLA-DRB1*0701, HLA-DRB1*1101, HLA-DRB1*1501 (DR2b).

A cut-off prediction score of 20 was used, wherein HLA binding strength increases with the prediction score. SYFPEITHI produced the following predicted epitopes:

**Table 2: SYFPEITHI search results**

| **Peptide sequence** | **Prediction score (SYFPEITHI)** |
|---|---|
| KKSLVRLSSCVPVAL (SEQ ID NO: 17) | 31 (HLA-DRB1*0101) |
| | 20 (HLA-DRB1*0401) |
| VALMSAMTTSSSQKN (SEQ ID NO: 18) | 28 (HLA-DRB1*0101) |
| | 20 (HLA-DRB1*0401) |
| PVALMSAMTTSSSQK (SEQ ID NO: 19) | 23 (HLA-DRB1*0101) |
| | 26 (HLA-DRB1*0401) |
| | 22 (HLA-DRB1*0701) |
| KCIMKEKKSLVRLSS (SEQ ID NO: 20) | 24 (HLA-DRB1*1501) |
| CVPVALMSAMTTSSS (SEQ ID NO: 21) | 23 (HLA-DRB1*0101) |
| | 24 (HLA-DRB1*1501) |
| LVRLSSCVPVALMSA (SEQ ID NO: 22) | 22 (HLA-DRB1*0101) |
| | 21 (HLA-DRB1*0301) |
| | 24 (HLA-DRB1*1501) |
| MKEKKSLVRLSSCVP (SEQ ID NO: 23) | 22 (HLA-DRB1*1101) |
| KSLVRLSSCVPVALM (SEQ ID NO: 24) | 20 (HLA-DRB1*0701) |
| | 20 (HLA-DRB1*1501) |
| SSCVPVALMSAMTTS (SEQ ID NO: 25) | 20 (HLA-DRB1*0401) |
| | 22 (HLA-DRB1*0701) |

As a result, SYFPEITHI predicted the consensus sequence:
KCIMKEKKSLVRLSSCVPVALMSAMTTSSSQKN (SEQ ID NO: 3).

### Example 3

The consensus sequences of Example 1 and 2 were compared and an optimised consensus sequence was produced.

**Table 3: Comparison of predicted consensus sequences**

| | |
|---|---|
| Example 1 predicted consensus peptide | SPKCIMKEKKSLVRLSSCVPVALMSAMTTSSSQ (SEQ ID NO: 16) |
| Example 2 predicted consensus peptide | KCIMKEKKSLVRLSSCVPVALMSAMTTSSSQKN (SEQ ID NO: 3) |
| Optimised consensus peptide | KCIMKEKKSLVRLSSCVPVALMSAMTTSSSQKN (SEQ ID NO: 3) |

### Example 4

The optimised consensus peptide (SEQ ID NO: 3) was modified in order to overcome predicted difficulties with synthetic production and use as a vaccine. In particular, the optimised consensus sequence (SEQ ID NO: 3) is 33 amino acids long, and peptides of this length are difficult to produce synthetically to the appropriate quality and yield. The two cysteine residues in the same peptide create problems with the stability and quality of the peptide due to peptide cyclisation by formation of intra- and inter-molecular disulphide bonds. This peptide cyclisation may also reduce immunological potency of the peptide, for example by impairing effective antigen processing. This may potentially cause processing of unrelated T-cell epitopes. In addition, the peptide cyclisation may induce unwanted inflammatory side effects, for example, antibody formation or allergic reactions. Furthermore, the eight amino acids at the N-terminal of the optimised consensus sequence (SEQ ID NO: 3) correspond to the wild-type TGFβR2, such that there is a risk of activating wild-type cross-reactive T-cells. Consequently, the optimised consensus sequence (SEQ ID NO: 3) was further modified to overcome these issues, and the peptides shown in Table 4 were designed. Figure 1 shows how the designed peptides relate to one another, as well as to the predicted and optimised consensus sequences shown in Table 3.

**Table 4: Modified optimised peptides**

| **Peptide Name** | **Sequence** |
|---|---|
| Fsp1 (SEQ ID NO: 4) | KCIMKEKKSLVRLSSCVPVALMSA |
| Fsp2 (SEQ ID NO: 5) | KGIMKEKKSLVRLSSCVPVALMSA |
| Fsp3 (SEQ ID NO: 6) | SSCVPVALMSAMTTSSSQKN |
| Fsp4 (SEQ ID NO: 7) | SSGVPVALMSAMTTSSSQKN |
| Fsp5 (SEQ ID NO: 3) | KCIMKEKKSLVRLSSCVPVALMSAMTTSSSQKN |

### Example 5

### i) Peptide Synthesis

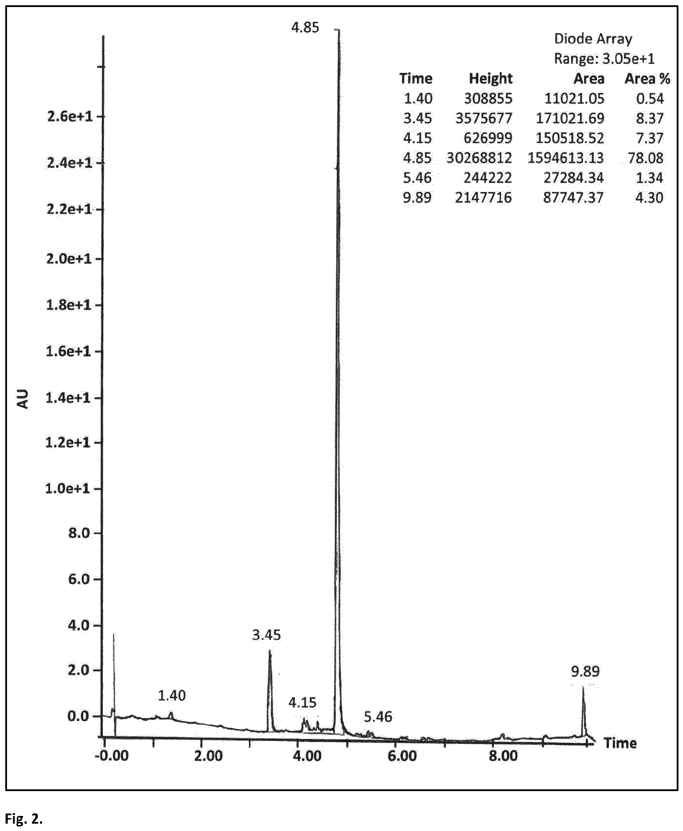

A batch of fsp5 (SEQ ID NO: 3) was prepared by solid phase peptide synthesis (SPPS) by using FMOC chemistry and Prelude synthesizer (Gyros Protein Technologies Inc., USA). The crude peptide was analysed by UPLC and MS. The desired peptide was observed by MS, and UPLC showed a purity of around 75% as demonstrated in Figure 2. UPLC system: Column; Acquity UPLC BEH C18 1.7mm, 21x150 mm, detection; PDA 210-500 nm, solvent A); 0.1% TFA in water, solvent B; 0.1% TFA in MeCN, gradient: 20-70% B (2-10 minutes, linear).

### ii) Peptide solubility and purification

The peptide was difficult to dissolve and it was attempted to dissolve the crude fsp5 (SEQ ID NO: 3) (-150 mg) in approximately 4 ml 50% MeCN in water under gentle heating. However, no clear solution could be obtained. Nevertheless, it was attempted to purify the crude solution by preparative HPLC (15-40% MeCN in water) after filtration of the crude suspension, which resulted in large losses of material. Only very small amounts of the desired peptide were obtained, and analysis by UPLC found that the peptide was impure.

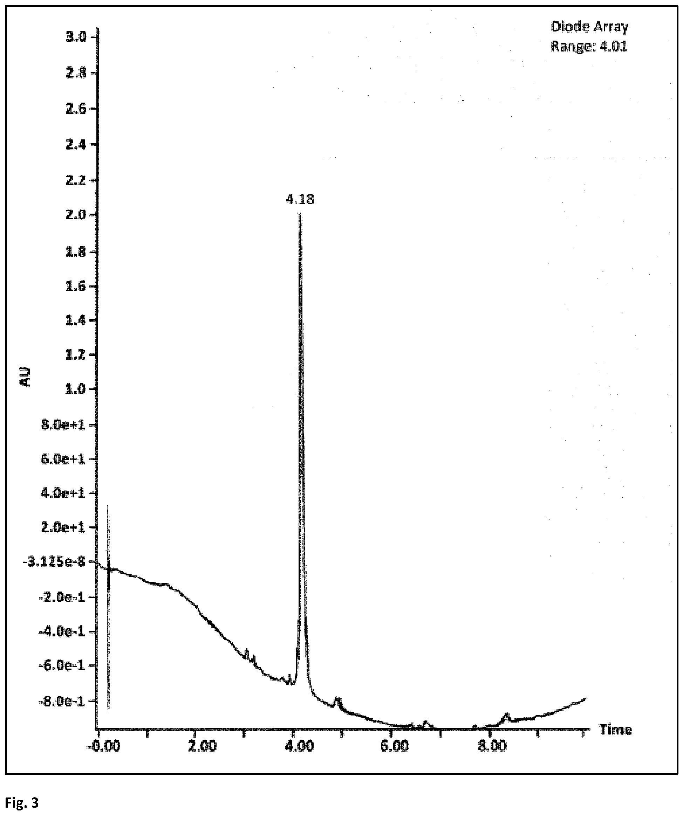

Another batch of fsp5 (SEQ ID NO: 3) was prepared, dissolved in neat DMSO under gentle heating and purified by preparative HPLC. The purity of the peptide was measured by UPLC, and was found to be about 90%, as shown in Figure 3. Again, only very small amounts of purified fsp5 (SEQ ID NO: 3) were obtained. It was noted that the addition of even small amounts of water led to precipitation of the peptide in the crude DMSO solution, so it is very likely that fsp5 (SEQ ID NO: 3) precipitates on the column during HPLC purification.

### iii) Peptide purity and stability

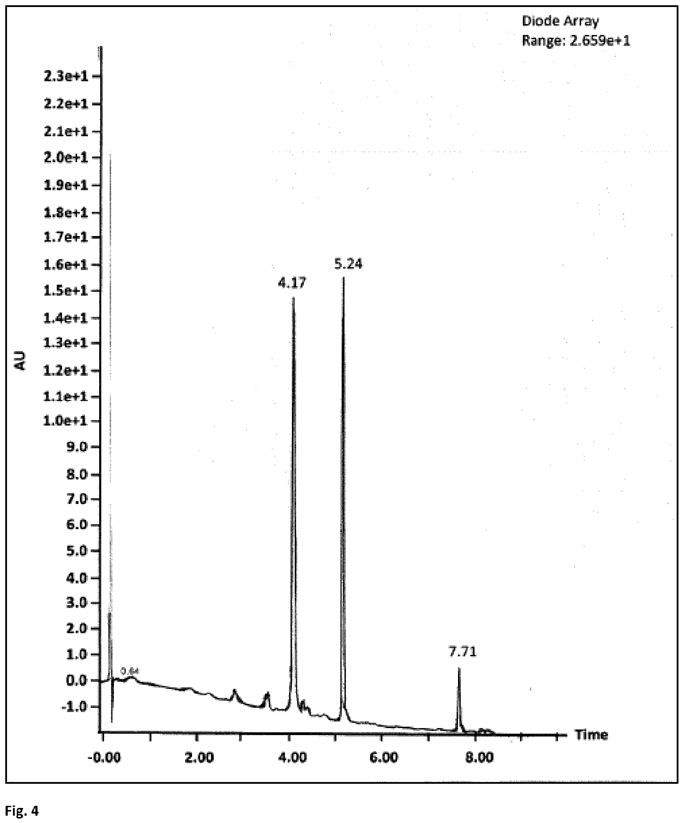

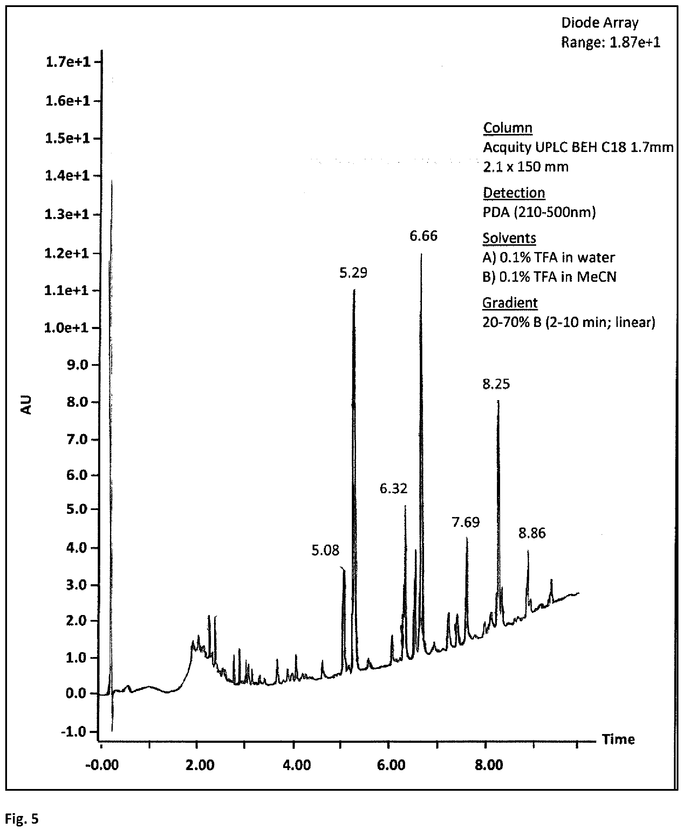

When the purity of the small amount of purified fsp5 (SEQ ID NO: 3) was reassessed by UPLC after lyophilisation, the purity had dropped from around 90% to below 50% as shown in Figure 4. The same trend was observed when the purity of crude fsp5 (SEQ ID NO: 3) was reassessed after a short storage (3 days) at room temperature followed by lyophilization, as demonstrated by Figure 5.

In summary, Example 5 shows that fsp5 (SEQ ID NO: 3) can be synthesized but is very difficult to produce in amounts sufficient for any practical purposes, and to a quality necessary for use in medicine, for example, as a constituent of a potential cancer vaccine.

### Example 6

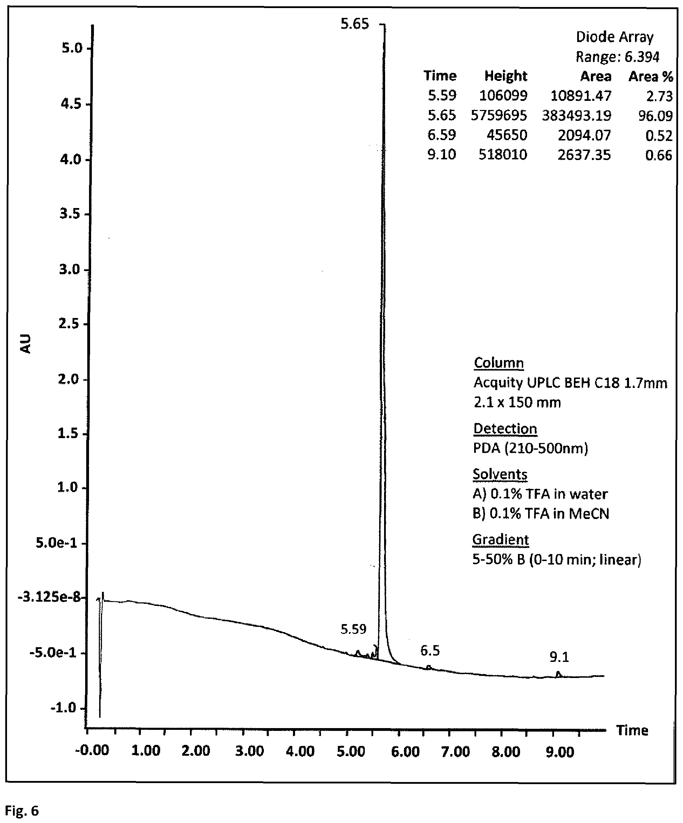

The Peptides fsp1 (SEQ ID NO: 4), fsp2 (SEQ ID NO: 5), fsp3 (SEQ ID NO: 6) and fsp4 (SEQ ID NO: 7), were synthesised by using SPPS and purified by HPLC as described above. The peptides were lyophilised after purification. The purity of each of the peptides was measured by UPLC and the UPLC traces are set out in Figures 6-9. UPLC system: Column; Acquity UPLC BEH C18 1.7mm, 21x150 mm, detection; PDA 210-500 nm, solvent A); 0.1% TFA in water, solvent B; 0.1% TFA in MeCN, gradient for fsp1 and fsp2: 5-50% B (0-10 minutes, linear), gradient for fsp3 and fsp4: 20-70% B (2-10 minutes, linear).
- Figure 6 show that the obtained purity for purified and lyophilised fsp1 (SEQ ID NO: 4) was 96%.

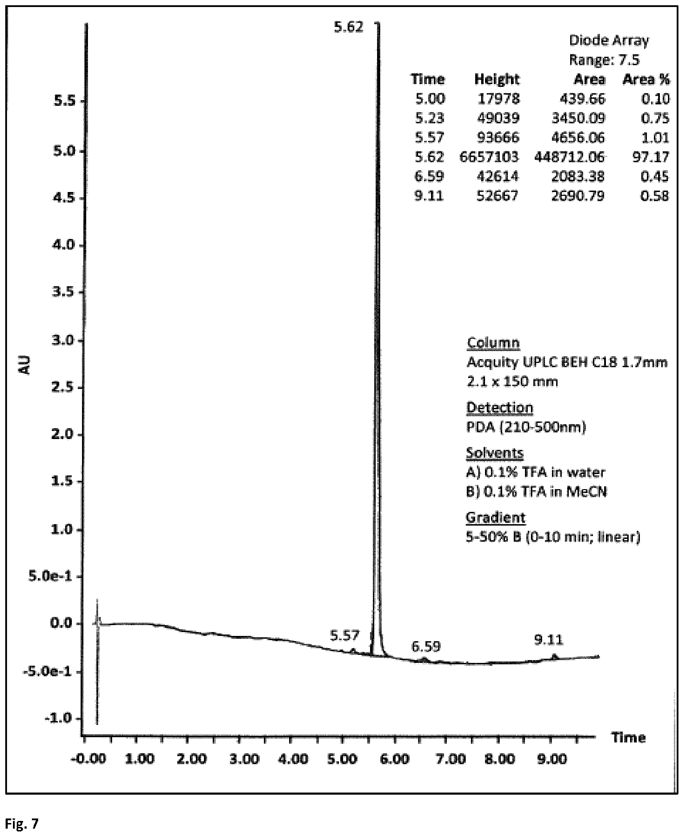

- Figure 7 show that the obtained purity for purified and lyophilised fsp2 (SEQ ID NO: 5) was 97%.
- Figure 8 show that the obtained purity for purified and lyophilised fsp3 (SEQ ID NO: 6) was 94.8%.
- Figure 9 show that the obtained purity for purified and lyophilised fsp4 (SEQ ID NO: 7) was 91.9%.

Correct molecular weight (MW) was confirmed for all four peptides by ESI-QTOF-MS (Table 5, Example 7).

In summary, Example 6 demonstrates that the peptides fsp1 (SEQ ID NO: 4), fsp2 (SEQ ID NO: 5), fsp3 (SEQ ID NO: 6) and fsp4 (SEQ ID NO: 7) can be produced without the chemical problems seen with fsp5 (SEQ ID NO: 3). It is therefore feasible to produce these shorter peptides for potential use as vaccines to induce peptide specific T cells.

### Example 7

The immunogenicity of each of the peptides of Table 4 was tested.

### i) Materials

**Table 5: Materials**

| **Peptide** | **Purity** | **Sequence** | **MW** | **Salt** | **MW incl.salt** |
|---|---|---|---|---|---|
| fsp1 | 96% | KCIMKEKKSLVRLSSCVP VALMSA (SEQ ID NO: 4) | 2622.34 | 6TFA | 3306.46 |
| fsp2 | 97% | KGIMKEKKSLVRLSSCVP VALMSA (SEQ ID NO: 5) | 2576.25 | 6TFA | 3260.37 |
| fsp3 | 94.8% | SSCVPVALMSAMTTSSS QKN (SEQ ID NO: 6) | 2029.36 | 2TFA | 2257.40 |
| fsp4 | 91.9% | SSGVPVALMSAMTTSSS QKN (SEQ ID NO: 7) | 1983.27 | 2TFA | 2211.38 |
| fsp5 | - 50% | KCIMKEKKSLVRLSSCVP VALMSAMTTSSSQKN (SEQ ID NO: 3) | 3571.21 | 7TFA | 4369.39 |

Fresh buffy coats from four healthy donors were obtained from a blood bank.

### ii) First in vitro stimulation of PBMC and T-cell proliferation

### Day 1:

### PBMC:

Peripheral blood mononuclear cells (PBMC) isolated from fresh buffy coats from four healthy donors were counted and suspended in DC medium to 15*10⁶ cells/ml, and subsequently diluted with DC-medium to 4*10⁶ cells/ml (Table 6).

**Table 6: Cell suspension and dilution**

| | | | | Cell suspension 15*10⁶ cells/ml | | Dilution to 4*10⁶ cells/ml | |
|---|---|---|---|---|---|---|---|
| Donor | Counted cells (*10⁶) | Viability % | Dilution | Total cells (*10⁶) | DC medium | Cell suspension | DC-medium |
| 1 | 1.85 | 96 | 5x | 462 | 30.8 ml | 6.7 ml | 18.3 ml |
| 2 | 2.49 | 78 | 5x | 622 | 41.4 ml | 6.7 ml | 18.3 ml |
| 3 | 2.24 | 94 | 5x | 560 | 37.3 ml | 6.7 ml | 18.3 ml |
| 4 | 2.25 | 93 | 5x | 563 | 37.5 ml | 6.7 ml | 18.3 ml |

| | | | | | | | |
|---|---|---|---|---|---|---|---|
| DC- medium: 500 ml CellGro DC medium (CellGenix) + 0.63 ml of 40mg/ml Gensumycin + 5 ml 1M HEPES buffer + 4ml of 200mg/ml Mucomyst/NAC | | | | | | | |

### In-vitro stimulation:

Four 24-well plates were used for each donor, with 4*10⁶ cells per well (each well has a volume of 1 ml).

Peptide cocktail: Solution of peptides fsp2+fsp4, containing 10µM of each peptide. 40µl peptide solution was added to each well.

The plates were incubated in a cell incubator for 14 days (37 °C, 5% CO₂). IL-2 and IL-7 were added on day 3. The cells were inspected daily.

### Day 14 - T cell Proliferation:

Test peptides: fsp2+fsp4 (SEQ ID NOs: 5 and 7), fsp1 (SEQ ID NO: 4), fsp2 (SEQ ID NO: 5), fsp3 (SEQ ID NO: 6), fsp4 (SEQ ID NO: 7), fsp5 (SEQ ID NO: 3).
Positive control: SEC3
Negative controls: T cells, T cells+APC (without addition of test peptides)
Mock: DMSO in PBS

### Plate set up for T cell proliferation:

Cells and reagents were added to plate wells (in triplicate) as described in Tables 7 and 8 below. The total volume added to each well was 0.20ml.
- T cells (PBMC): 50 000 cells /well (0.25*10⁶ cells/ml)
- APC: Irradiated (30Gy, 8 minutes) autologous feeder cells: 50 000 cells /well
- Peptide (fspx): 0.2nmol peptide/well (each peptide), concentration: 10µM peptide. (fsp was dissolved in DMSO before dilution to correct concentration)

**Table 7: Plate 1 (96 wells) set up:**

| **Row A-H** | **Wells 1-3** | **Wells 4-6** | **Wells 7-9** | **Wells 10-12** |
|---|---|---|---|---|
| A | DC-medium | DC-medium | DC-medium | DC-medium |
| B (donor 1) | T cells | T cells + APC | T cells+ APC+fsp1 | T cells+ APC+fsp2 |
| C (donor 1) | T cells+ APC+fsp3 | T cells+ APC+fsp4 | T cells+ APC+fsp5 | T cells+ APC+fsp2+fsp4 |
| D (donor 1) | T cells+ APC+mock | T cells+ APC+SEC3 | medium | medium |
| E (donor 2) | T cells | T cells + APC | T cells+ APC+fsp1 | T cells+ APC+fsp2 |
| F (donor 2) | T cells+ APC+fsp3 | T cells+ APC+fsp4 | T cells+ APC+fsp5 | T cells+ APC+fsp2+fsp4 |
| G (donor 2) | T cells+ APC+mock | T cells+ APC+SEC3 | medium | medium |
| H | medium | medium | medium | medium |

**Table 8: Plate 2 (96 wells) set up:**

| **Row A-H** | **well 1-3** | **well 4-6** | **well 7-9** | **well 10-12** |
|---|---|---|---|---|
| A | DC-medium | DC-medium | DC-medium | DC-medium |
| B (donor 3) | T cells | T cells + APC | T cells+ APC+fsp1 | T cells+ APC+fsp2 |
| C (donor 3) | T cells+ APC+fsp3 | T cells+ APC+fsp4 | T cells+ APC+fsp5 | T cells+ APC+fsp2+fsp4 |
| D (donor 3) | T cells+ APC+mock | T cells+ APC+SEC3 | medium | medium |
| E (donor 4) | T cells | T cells + APC | T cells+ APC+fsp1 | T cells+ APC+fsp2 |
| F (donor 4) | T cells+ APC+fsp3 | T cells+ APC+fsp4 | T cells+ APC+fsp5 | T cells+ APC+fsp2+fsp4 |
| G (donor 4) | T cells+ APC+mock | T cells+ APC+SEC3 | medium | medium |
| H | medium | medium | medium | medium |

The plates were incubated in a cell incubator for 48 hours (37 °C, 5% CO₂).

### Day 16.

After incubation for 48 hours, 20µl 3H-tThymidine solution (5µCi/ml) was added to each well and the plates were incubated for approximately 17 hours (37 °C, 5% CO₂).

### Day 17.

After 17 hours the cells were harvested (Unifilters) and dried on the filters at 45°C until completely dry. After covering the bottom of the Unifilters with adhesive covers (delivered with the Unifilters) 25 µl micro scintillation liquid was added to each well, the plate was covered with TopSeal and 3H-Thymidine uptake was measured as counts per minute (CPM) using a microplate scintillation counter.

### Proliferation results:

The T cell proliferation results after the first round of *in vitro* stimulation with the cocktail of fsp2 and fsp4 peptides are presented as stimulation index (SI) in Figure 10. Sl= mean CPM(triplicates) of (T cells+APC+test peptide(s)) divided by mean CPM (triplicates) of (T cells+APC). SI ≥ 2 is a clear signal of immunogenicity.

### iii) In vitro re-stimulation of PBMC and T-cell proliferation

PBMCs harvested after the first *in vitro* stimulation were re-stimulated *in vitro* according to the protocol set out above, with 2×10⁶ cells per well. T-cell proliferation was tested according to the protocol set out above.

### Proliferation results:

The T cell proliferation results after a second round of *in vitro* stimulation with the cocktail of fsp2 and fsp4 peptides are presented as stimulation index (SI) in Figure 11.

Thus, Figures 10 and 11 show that the peptides fsp2 and fsp4, having an amino acid substitution, are immunogenic and can activate T-cells, and that the activated T-cells are cross-reactive for peptides of the naturally occurring -1a frameshifted TGFβ2 protein. In addition, Figures 10-12 show that fsp1 and fsp5, which are peptides of the naturally-occurring -1a frameshifted TGFβ2 protein, stimulated T-cells induced from PMBCs and, therefore, are immunogenic. Consequently, the modified peptides fsp2 (SEQ ID NO. 5) and fsp4 (SEQ ID NO. 7), as well as unmodified peptides fsp1 (SEQ ID NO: 4) and fsp5 (SEQ ID NO: 3), can be used to stimulate induction of TGFβR2 frameshift mutant specific T-cells.

### Example 8

The same protocol as that set out in Example 7 was used to test the immunogenicity of fsp6 and fsp7 (SEQ ID NO: 26 and SEQ ID NO: 27, respectively), except for the peptide mixture used to induce T-cells from PMBCs from fresh buffy coats from four healthy donors. The peptide mixture used in this Example to induce T-cells from PMBCs was made up of fsp2 (SEQ ID NO: 5; 10µM), fsp8 (SEQ ID NO: 31; 10µM) and fsp9 (SEQ ID NO: 32; 10µM). Fsp8 (SEQ ID NO: 31; RNRIPAVLRTEGEPLHTPSVGMRET) is a peptide of ASTE1 having a -1a frameshift mutation, and fsp9 (SEQ ID NO: 32; KTILKKAGIGMCVKVSSIFFINKQK) is a peptide of TAF1β having a -1a frameshift mutation.

At day 14 after induction, a T-cell proliferation assay was carried out as in Example 7, using fsp2 (SEQ ID NO: 5), fsp6 (SEQ ID NO: 26) and fsp7 (SEQ ID NO: 27) as test peptides. As fsp8 and fsp9 have very different amino acid sequences from fsp2, only fsp2 would be able to induce T-cells which are capable of recognising and being stimulated by fsp2, fsp6 and fsp7. In particular, as shown in Figure 11, fsp6 and fsp7 are truncated versions of fsp2. Two further rounds of T-cell stimulation were also carried out, with a T-cell proliferation assay being carried out 14 days after each stimulation.

The T-cell proliferation results after the first round of *in vitro* stimulation are presented as stimulation index (SI) in Figure 13. In particular, Figure 13 shows that fsp6 is capable of activating T-cells, such that fsp6 is immunogenic and can be used as a vaccine or treatment against cancer. Figure 13 also shows that T-cells induced by fsp2 (SEQ ID NO: 5) are cross-reactive for peptides of the naturally-occurring -1a frameshifted protein which are shorter than fsp2 (SEQ ID NO: 5), i.e. fsp6 (SEQ ID NO: 26). It is also expected, in view of the results shown in Figures 11 and 12, that a peptide having the sequence of fsp6 (SEQ ID NO: 26) but having an amino acid substitution corresponding to that in fsp2, and particularly a C-to-G amino acid substitution as in fsp2, is also immunogenic and is useful as a vaccine or treatment against cancer. In particular, as mentioned in Example 7, Figure 11 shows that T-cells induced by fsp2 (SEQ ID NO: 5), which has a C-to-G amino acid substitution, are cross-reactive for peptides of the naturally-occurring -1a frameshifted protein, such that the C-to-G amino acid substitution is immunologically acceptable. Thus, it is expected that fsp6a (SEQ ID NO: 28), which is identical to fsp6 (SEQ ID NO: 26) except for the same C-to-G amino acid substitution as fsp2 (SEQ ID NO: 5), will also be immunogenic.

Furthermore, Figure 13 shows that fsp6 (SEQ ID NO: 26) is less immunogenic than fsp2 (SEQ ID NO: 5), but that both fsp6 and fsp2 are much more immunogenic than fsp7 (SEQ ID NO: 27). Fsp6 has the same amino acid sequence as fsp2, except that fsp6 is truncated at the C-terminus compared to fsp2 and does not have a C-to-G amino acid substitution. Fsp7 has the same amino acid sequence as fsp2 but is truncated at the N-terminus compared to fsp2. In view of the difference in immunogenicity between these three peptides, shown in Figure 13, it is expected that the addition of at least one additional amino acid at the C-terminus of fsp6, up to the full length of fsp2, will provide the same immunogenic activity of fsp2. In addition, it is expected that at least one additional amino acid at the N-terminus of fsp7 is required in order to increase its immunogenicity and for the peptide to comprise immunologically effective epitopes. Consequently, it is expected that the 9-mer peptide LVRLSSCVP (fsp1a; SEQ ID NO: 29) is immunogenic, as this peptide comprises a sequence shared by fsp6 and fsp7, with the addition of one amino acid at the C-terminus compared to fsp6 and one amino acid at the N-terminus compared to fsp7. Furthermore, as the C-to-G amino acid substitution has been shown to be immunologically acceptable, it is expected that the 9-mer peptide having a C-toG amino acid substitution (fsp1b; SEQ ID NO: 30) will also be immunogenic.

## Claims

1. A peptide comprising a fragment of SEQ ID NO: 8, wherein the fragment comprises at least 8 consecutive amino acids of SEQ ID NO: 8, including at least one of positions 121 and 135 of SEQ ID NO: 8, wherein the peptide is capable of inducing an immune response against a TGFβR2 -1a frameshift mutant protein.

2. A peptide according to claim 1, wherein the fragment comprises at least 9, preferably at least 12, consecutive amino acids of SEQ ID NO: 8, including at least one of positions 121 and 135 of SEQ ID NO: 8, and/or wherein the peptide comprises no more than 33 amino acids, preferably no more than 24 amino acids, preferably no more than 20 amino acids, preferably no more than 17 amino acids or preferably no more than 9 amino acids.

3. A peptide according to claim 1 or 2, wherein the fragment comprises positions 121 to 132 of SEQ ID NO: 8, positions 129 to 137 of SEQ ID NO: 8 or positions 135 to 146 of SEQ ID NO: 8.

4. A peptide according to any one of the preceding claims, wherein the amino acid corresponding to position 121 or 135 of SEQ ID NO: 8 is glycine.

5. A peptide according to any one of the preceding claims, wherein the peptide comprises the amino acid sequence of SEQ ID NO: 5, SEQ ID NO: 7, SEQ ID NO: 28 or SEQ ID NO: 30, preferably wherein the peptide consists of SEQ ID NO: 5, SEQ ID NO: 7, SEQ ID NO: 28 or SEQ ID NO: 30.

6. A nucleic acid molecule encoding the peptide as defined in any one of claims 1 to 5.

7. A peptide mixture comprising a first and a second peptide, wherein the first and second peptides are, independently, peptides as defined in any one of claims 1 to 5, and wherein the first peptide is different from the second peptide.

8. A vector comprising a nucleic acid molecule comprising a nucleotide sequence which encodes a peptide as defined in any one of claims 1-5 or a peptide mixture as defined in claim 7.

9. A host cell comprising a vector as defined in claim 8.

10. A non-transfected T-cell specific for a peptide as defined in any one of claims 1 to 5.

11. A T-cell mixture comprising non-transfected T-cells specific for a peptide mixture as defined in claim 7.

12. A pharmaceutical composition comprising a peptide as defined in any one of claims 1 to 5, a nucleic acid molecule as defined in claim 6, a peptide mixture as defined in claim 7, a vector as defined in claim 8, a host cell as defined in claim 9, a non-transfected T-cell as defined in claim 10 or a T-cell mixture as defined in claim 11, and pharmaceutically-acceptable carrier, diluent or excipient.

13. A peptide as defined in any one of claims 1 to 5, a nucleic acid molecule as defined in claim 6, a peptide mixture as defined in claim 7, a vector as defined in claim 8, a host cell as defined in claim 9, a T-cell as defined in claim 10, a T-cell mixture as defined in claim 11 or a pharmaceutical composition as defined in claim 12, for use in the treatment and/or prophylaxis of cancer, preferably wherein the cancer is colorectal cancer or stomach cancer.

14. A method of selecting a peptide, nucleic acid molecule, vector, host cell, peptide mixture, T-cell, T-cell mixture or a pharmaceutical composition for administration to a patient, comprising:
i) identifying whether a cancer patient has a frameshift mutation in the TGFβR2 protein and, if so,
ii) selecting a peptide as defined in any one of claims 1 to 5, a nucleic acid molecule as defined in claim 6, a peptide mixture as defined in claim 7, a vector as defined in claim 8, a host cell as defined in claim 9, a T-cell as defined in claim 10, a T-cell mixture as defined in claim 11 or a pharmaceutical composition as defined in claim 12.

## Patentansprüche

1. Peptid, umfassend ein Fragment der SEQ ID Nr. 8, wobei das Fragment mindestens 8 aufeinanderfolgende Aminosäuren der SEQ ID Nr. 8 umfasst, einschließlich mindestens einer der Positionen 121 und 135 der SEQ ID Nr. 8, wobei das Peptid in der Lage ist, eine Immunreaktion gegen ein TGFβR2-1a-Frameshift-mutiertes Protein zu induzieren.

2. Peptid nach Anspruch 1, wobei das Fragment mindestens 9, vorzugsweise mindestens 12, aufeinanderfolgende Aminosäuren der SEQ ID Nr. 8 umfasst, einschließlich mindestens einer der Positionen 121 und 135 der SEQ ID Nr. 8, und/oder wobei das Peptid nicht mehr als 33 Aminosäuren, vorzugsweise nicht mehr als 24 Aminosäuren, vorzugsweise nicht mehr als 20 Aminosäuren, vorzugsweise nicht mehr als 17 Aminosäuren oder vorzugsweise nicht mehr als 9 Aminosäuren umfasst

3. Peptid nach Anspruch 1 oder 2, wobei das Fragment die Positionen 121 bis 132 der SEQ ID Nr. 8, die Positionen 129 bis 137 der SEQ ID Nr. 8 oder die Positionen 135 bis 146 der SEQ ID Nr. 8 umfasst.

4. Peptid nach einem der vorhergehenden Ansprüche, wobei die Aminosäure, die der Position 121 oder 135 der SEQ ID Nr. 8 entspricht, Glycin ist.

5. Peptid nach einem der vorhergehenden Ansprüche, wobei das Peptid die Aminosäuresequenz der SEQ ID NO 5, SEQ ID Nr. 7, SEQ ID Nr. 28 oder SEQ ID Nr. 30 umfasst, wobei das Peptid vorzugsweise aus SEQ ID NO 5, SEQ ID NO 7, SEQ ID Nr. 28 oder SEQ ID Nr. 30 besteht.

6. Nukleinsäuremolekül, das für das in einem der Ansprüche 1 bis 5 definierte Peptid codiert.

7. Peptidgemisch, umfassend ein erstes und ein zweites Peptid, wobei das erste und das zweite Peptid unabhängig voneinander Peptide sind, wie sie in einem der Ansprüche 1 bis 5 definiert sind, und wobei sich das erste Peptid von dem zweiten Peptid unterscheidet.

8. Vektor, umfassend ein Nukleinsäuremolekül, das eine Nukleotidsequenz umfasst, die für ein Peptid, wie in einem der Ansprüche 1 bis 5 definiert, oder ein Peptidgemisch, wie in Anspruch 7 definiert, codiert.

9. Wirtszelle, umfassend einen Vektor gemäß Anspruch 8.

10. Nicht-transfizierte T-Zelle, die für ein Peptid, wie in einem der Ansprüche 1 bis 5 definiert, spezifisch ist.

11. T-Zell-Mischung, umfassend nicht-transfizierte T-Zellen, die für eine Peptidmischung, wie nach Anspruch 7 definiert, spezifisch sind.

12. Pharmazeutische Zusammensetzung, umfassend ein Peptid, wie in einem der Ansprüche 1 bis 5 definiert, ein Nukleinsäuremolekül, wie in Anspruch 6 definiert, ein Peptidgemisch, wie in Anspruch 7 definiert, einen Vektor, wie in Anspruch 8 definiert, eine Wirtszelle, wie in Anspruch 9 definiert, eine nicht-transfizierte T-Zelle, wie in Anspruch 10 definiert, oder ein T-Zellgemisch, wie in Anspruch 11 definiert, und einen pharmazeutisch annehmbaren Träger, ein pharmazeutisch annehmbares Verdünnungsmittel oder einen pharmazeutisch annehmbaren Hilfsstoff.

13. Peptid, wie in einem der Ansprüche 1 bis 5 definiert, Nukleinsäuremolekül, wie in Anspruch 6 definiert, Peptidgemisch, wie in Anspruch 7 definiert, Vektor, wie in Anspruch 8 definiert, Wirtszelle, wie in Anspruch 9 definiert, T-Zelle, wie in Anspruch 10 definiert, T-Zellgemisch, wie in Anspruch 11 definiert oder pharmazeutische Zusammensetzung, wie in Anspruch 12 definiert, zur Verwendung bei der Behandlung und/oder Prophylaxe von Krebs, wobei der Krebs vorzugsweise Kolorektalkrebs oder Magenkrebs ist.

14. Verfahren zur Auswahl eines Peptids, eines Nukleinsäuremoleküls, eines Vektors, einer Wirtszelle, einer Peptidmischung, einer T-Zelle, einer T-Zellmischung oder einer pharmazeutischen Zusammensetzung zur Verabreichung an einen Patienten, Folgendes umfassend:
i) Identifizieren, ob ein Krebspatient eine Frameshift-Mutation im TGFβR2-Protein besitzt und, falls ja
ii) Auswählen eines Peptids, wie in einem der Ansprüche 1 bis 5 definiert, eines Nukleinsäuremoleküls, wie in Anspruch 6 definiert, einer Peptidmischung, wie in Anspruch 7 definiert, eines Vektors, wie in Anspruch 8 definiert, einer Wirtszelle, wie in Anspruch 9 definiert, einer T-Zelle, wie in Anspruch 10 definiert, einer T-Zellmischung, wie in Anspruch 11 definiert, oder einer pharmazeutischen Zusammensetzung, wie in Anspruch 12 definiert.

## Revendications

1. Peptide comprenant un fragment de la SEQ. ID N°8, dans lequel le fragment comprend au moins 8 acides aminés consécutifs de la SEQ. ID N°8, incluant au moins une des positions 121 et 135 de la SEQ. ID N°8, dans lequel le peptide est capable d'induire une réponse immunitaire contre une protéine mutante par décalage TGFβR2-1a.

2. Peptide selon la revendication 1, dans lequel le fragment comprend au moins 9, de préférence au moins 12 acides aminés consécutifs de la SEQ. ID N°8, incluant au moins une des positions 121 et 135 de la SEQ. ID N°8, et/ou dans lequel le peptide comprend au plus 33 acides aminés, de préférence au plus 24 acides aminés, de préférence au plus 20 acides aminés, de préférence au plus 17 acides aminés ou de préférence au plus 9 acides aminés.

3. Peptide selon la revendication 1 ou 2, dans lequel le fragment comprend les positions 121 à 132 de la SEQ. ID N°8, les positions 129 à 137 de la SEQ. ID N°8 ou les positions 135 à 146 de la SEQ. ID N°8.

4. Peptide selon l'une quelconque des revendications précédentes, dans lequel l'acide aminé correspondant à la position 121 ou 135 de la SEQ. ID N°8 est la glycine.

5. Peptide selon l'une quelconque de revendications précédentes, dans lequel le peptide comprend la séquence d'acide aminé de la SEQ. ID N°5, SEQ. ID N°7, SEQ. ID N°28 ou SEQ. ID N°30, de préférence dans lequel le peptide consiste en SEQ. ID N°5, SEQ. ID N°7, SEQ. ID N°28 ou SEQ. ID N°30.

6. Molécule d'acide nucléique codante pour le peptide tel que défini dans l'une quelconque des revendications 1 à 5.

7. Mélange peptidique comprenant un premier et un second peptide, dans lequel le premier et le second peptide sont, indépendamment, des peptides tels que définis dans l'une quelconque des revendications 1 à 5, et dans lequel le premier peptide est différent du second peptide.

8. Vecteur comprenant une molécule d'acide nucléique comprenant une séquence nucléotidique qui est codante pour un peptide tel que défini dans l'une quelconque des revendications 1 à 5, ou un mélange peptidique tel que défini dans la revendication 7.

9. Cellule hôte comprenant un vecteur tel que défini dans la revendication 8.

10. Lymphocyte T non transfecté spécifique pour un peptide tel que défini dans l'une quelconque des revendications 1 à 5.

11. Mélange de lymphocytes T comprenant des lymphocytes T non transfectés spécifiques pour un mélange peptidique tel que défini dans la revendication 7.

12. Composition pharmaceutique comprenant un peptide tel que défini dans l'une quelconque des revendications 1 à 5, une molécule d'acide nucléique telle que définie dans la revendication 6, un mélange peptidique tel que défini dans la revendication 7, un vecteur tel que défini dans la revendication 8, une cellule hôte telle que définie dans la revendication 9, un lymphocyte T non-transfecté tel que défini dans la revendication 10 ou un mélange de lymphocytes T tel que défini dans la revendication 11, et un support, diluant ou excipient pharmaceutiquement acceptables.

13. Peptide tel que défini dans l'une quelconque des revendications 1 à 5, molécule d'acide nucléique telle que définie dans la revendication 6, mélange peptidique tel que défini dans la revendication 7, vecteur tel que défini dans la revendication 8, cellule hôte telle que définie dans la revendication 9, lymphocyte T tel que défini dans la revendication 10, mélange de lymphocytes T tel que défini dans la revendication 11 ou composition pharmaceutique telle que définie dans la revendication 12, pour utilisation dans le traitement et/ou la prophylaxie d'un cancer, de préférence dans lequel le cancer est un cancer colorectal ou un cancer de l'estomac.

14. Procédé de sélection d'un peptide, d'une molécule d'acide nucléique, d'un vecteur, d'une cellule hôte, d'un mélange peptidique, d'un lymphocyte T, d'un mélange de lymphocytes T ou d'une composition pharmaceutique pour administration à un patient, comprenant les étapes consistant à :
i) identifier si un patient souffrant de cancer présente une mutation par décalage dans la protéine TGFβR2 et dans l'affirmative,
ii) sélectionner un peptide tel que défini dans l'une quelconque des revendications 1 à 5, une molécule d'acide nucléique telle que définie dans la revendication 6, un mélange peptidique tel que défini dans la revendication 7, un vecteur tel que défini dans la revendication 8, une cellule hôte telle que définie dans la revendication 9, un lymphocyte T tel que défini dans la revendication 10, un mélange de lymphocytes T tel que défini dans la revendication 11 ou une composition pharmaceutique telle que définie dans la revendication 12.
